# EUROPÄISCHE PATENTSCHRIFT

(11) **EP 0 842 171 B1**
(45) Veröffentlichungstag und Bekanntmachung des Hinweises auf die Patenterteilung: **16.08.2000**
(21) Anmeldenummer: 96924878.0
(22) Anmeldetag: 04.07.1996
(51) Int. Cl.: C07D 405/12, C07D 409/12, C07D 401/12, C07D 403/12, A01N 47/38

(54) **HERBIZIDE SULFONYLAMINO(THIO)CARBONYLTRIAZOLINONE MIT HETEROCYCLYLOXY-SUBSTITUENTEN**
HERBICIDAL SULPHONYLAMINO(THIO)CARBONYL TRIAZOLINONES WITH HETEROCYCLYLOXY SUBSTITUENTS
SULFONYLAMINO(THIO)CARBONYLTRIAZOLINONES HERBICIDES AVEC SUBSTITUENTS HETEROCYCLYLOXY

(30) Priorität: 17.07.1995 DE 19525973
(43) Veröffentlichungstag der Anmeldung: 20.05.1998
(73) Patentinhaber: BAYER AG, 51368 Leverkusen (DE)
(72) Erfinder: MÜLLER, Klaus-Helmut, D-40593 Düsseldorf (DE); KLUTH, Joachim, D-40764 Langenfeld (DE); KIRSTEN, Rolf, D-40789 Monheim (DE); GESING, Ernst, Rudolf, D-40699 Erkrath (DE); FINDEISEN, Kurt, D-51375 Leverkusen (DE); JANSEN, Johannes, Rudolf, D-40789 Monheim (DE); KÖNIG, Klaus, D-51519 Odenthal (DE); DREWES, Mark, Wilhelm, D-40764 Langenfeld (DE); DOLLINGER, Markus, D-51381 Leverkusen (DE); SANTEL, Hans-Joachim, D-51371 Leverkusen (DE)
(86) Internationale Anmeldenummer: EP9602933
(87) Internationale Veröffentlichungsnummer: WO9703981

(56) Entgegenhaltungen:
- EP-A- 0 185 401
- EP-A- 0 507 171
- CHEMISCHE BERICHTE, Bd. 110, Nr. 5, 9.Mai 1977, WEINHEIM DE, Seiten 1716-29, XP002013585 H. WAMHOFF ET AL.: "Neue Addukte des 4-Phenyl-1,2,4-triazolin-3,5-dions sowie des Phthalazin-1,4-dions"

## Beschreibung

Die Erfindung betrifft neue Sulfonylamino(thio)carbonyltriazolinone mit Heterocyclyloxy-Substituenten, mehrere Verfahren und neue Zwischenprodukte zu ihrer Herstellung sowie ihre Verwendung als Herbizide.

Es ist bereits bekannt, daß bestimmte Sulfonylaminocarbonyltriazolinone herbizide Eigenschaften aufweisen (vgl. EP-A 341489, EP-A 422469, EP-A 425948, EP-A 431291, EP-A 507171). Aus der EP-A 185 401 ist ferner bekannt, daß sich herbizid wirksame 1,2,4-Triazole mit über Sauerstoff gebundenen Aryl- und Heteroarylsubstituenten herstellen lassen. Die Wirkung dieser Verbindungen ist jedoch nicht in allen Belangen zufriedenstellend.

Es wurden nun die neuen Sulfonylamino(thio)carbonyltriazolinone mit Heterocyclyloxy-Substituenten der allgemeinen Formel (I), 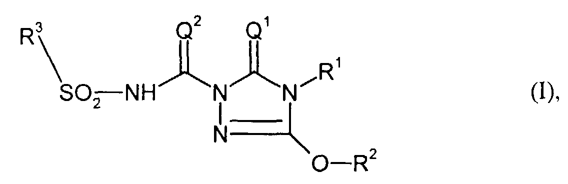 in welcher
- Q¹: für Sauerstoff steht,
- Q²: für Sauerstoff oder Schwefel steht,
- R¹: für Wasserstoff, Hydroxy, Amino, C₁-C₆-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für C₁-C₆-Alkyloxy oder C₃-C₆-Alkenyloxy, für jeweils gegebenenfalls durch Fluor und/oder Chlor substituiertes C₁-C₆-Alkylamino, Di-(C₁-C₄-alkyl)-amino oder C₁-C₄-Alkanoylamino, für jeweils gegebenenfalls durch Fluor, Chlor, Brom und/oder C₁-C₄-Alkyl substituiertes C₃-C₆-Cycloalkyl oder C₃-C₆-Cycloalkyl-C₁-C₄-alkyl, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl oder Phenyl-C₁-C₄-alkyl steht,
- R²: für Oxetanyl oder Tetrahydrofuryl steht und
- R³: für die Gruppierung 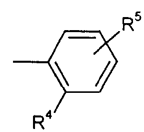 steht, worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxycarbonyl, C₁-C₄-Alkylaminocarbonyl, Di-(C₁-C₄-alkyl)-amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxy-carbonyl, C₁-C₄-Alkylthio, C₁-C₄-Alkylsulfinyl oder C₁-C₄-Alkylsulfonyl substituiert ist), für C₂-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxy-carbonyl substituiert ist), C₃-C₆-Alkinyloxy, C₃-C₆-Alkinylthio oder für den Rest -S(O)ₚ-R⁶ stehen, wobei
p für die Zahlen 1 oder 2 steht und
- R⁶: für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₁-C₄-Alkoxy-carbonyl, C₁-C₄-Alkylamino-carbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-Cycloalkyl-C₁-C₂-alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxy- carbonyl substituiert ist) steht,
- R⁴ und/oder R⁵: weiterhin für Phenyl oder Phenoxy, für C₁-C₄-Alkylcarbonyl-amino, C₁-C₄-Alkoxy-carbonylamino, C₁-C₄-Alkylamino-carbonyl-amino, Di-(C₁-C₄-alkyl)-amino-carbonylamino, oder für den Rest -CO-R⁸ stehen, wobei
R⁸ für Wasserstoff, C₁-C₆-Alkyl, C₃-C₆-Cycloalkyl, C₁-C₆-Alkoxy, C₃-C₆-Cycloalkoxy, C₃-C₆-Alkenyloxy, C₁-C₄-Alkylthio, C₁-C₄-Alkylamino, C₁-C₄-Alkoxyamino, C₁-C₄-Alkoxy-C₁-C₄-alkylamino oder Di-(C₁-C₄-alkyl)-amino steht (welche gegebenenfalls durch Fluor und/oder Chlor substituiert sind),
- R⁴ und/oder R⁵: weiterhin für Trimethylsilyl, Thiazolinyl, C₁-C₄-Alkylsulfonyloxy, Di-(C₁-C₄-alkyl)-aminosulfonylamino oder für den Rest
-CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenyloxy, C₃-C₆-Alkinyloxy oder Benzyloxy, für Amino, C₁-C₄-Alkylamino, Di-(C₁-C₄-alkyl)-amino, Phenylamino, C₁-C₄-Alkyl-carbonylamino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht,
weiterhin
- R³: für die Gruppierung 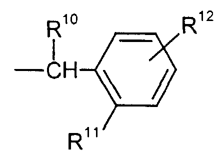 steht,
worin
R¹⁰ für Wasserstoff oder C₁-C₄-Alkyl steht,
R¹¹ und R¹² gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Carboxy, C₁-C₄-Alkoxycarbonyl, Dimethylaminocarbonyl, C₁-C₄-Alkylsulfonyl oder Di-(C₁-C₄-alkyl)-aminosulfonyl stehen;
weiterhin
- R³: für den Rest 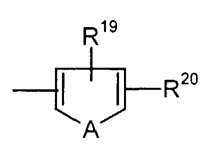 steht,
worin
R¹⁹ und R²⁰ gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), C₁-C₄-Alkylthio, C₁-C₄-Alkylsulfinyl oder C₁-C₄-Alkylsulfonyl (welche gegebenenfalls durch Fluor und/oder Chlor substituiert sind), Di-(C₁-C₄-alkyl)-amino-sulfonyl, C₁-C₄-Alkoxycarbonyl oder Dimethylaminocarbonyl stehen, und
A für Schwefel steht.

Gegenstand der Erfindung sind weiter vorzugsweise Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium-, Tri-(C₁-C₄-alkyl)-sulfonium-, C₅- oder C₆-Cycloalkyl-ammonium- und Di-(C₁-C₂-alkyl)-benzyl-ammonium-Salze von Verbindungen der Formel (I), in welcher Q¹, Q², R¹, R² und R³ die oben vorzugsweise angegebenen Bedeutungen haben.

Man erhält die neuen Sulfonylamino(thio)carbonyltriazolinone mit Heterocyclyloxy-Substituenten der allgemeinen Formel (I), wenn man
(a) Triazolinone der allgemeinen Formel (II) 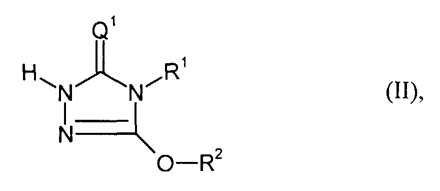 in welcher
   - Q¹, R¹ und R²: die oben angegebenen Bedeutungen haben,
   mit Sulfonyliso(thio)cyanaten der allgemeinen Formel (III)

   R³-SO₂-N=C=Q² (III)

   in welcher
   - Q² und R³: die oben angegebenen Bedeutungen haben,
   gegebenenfalls in Gegenwart eines Reaktionshilfsmittels und gegebenenfalls in Gegenwart eines Verdünnungsmittels umsetzt,
   oder wenn man
(b) Triazolinon-Derivate der allgemeinen Formel (IV) 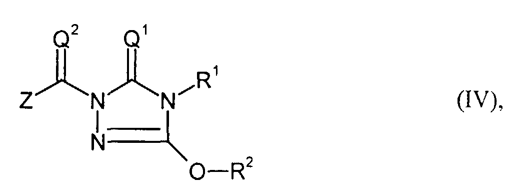 in welcher
   - Q¹, Q², R¹ und R²: die oben angegebenen Bedeutungen haben und
   - Z: für Fluor, Chlor, Brom, Methoxy, Ethoxy, Benzyloxy, Phenoxy, Halogen- oder Nitrophenoxy steht,
   mit Sulfonsäureamiden der allgemeinen Formel (V)

   R³-SO₂-NH₂ (V)

   in welcher
   - R³: die oben angegebene Bedeutung hat,
   gegebenenfalls in Gegenwart eines Säureakzeptors und gegebenenfalls in Gegenwart eines Verdünnungsmittels umsetzt,
   oder wenn man
(c) Triazolinone der allgemeinen Formel (II) 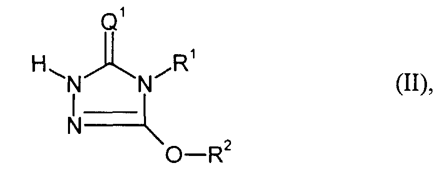 in welcher
   - Q¹, R¹ und R²: die oben angegebenen Bedeutungen haben,
   mit Sulfonsäureamid-Derivaten der allgemeinen Formel (VI)

   R³-SO₂-NH-CQ²-Z (VI)

   in welcher
   - Q² und R³: die oben angegebenen Bedeutungen haben und
   - Z: für Fluor, Chlor, Brom, Methoxy, Ethoxy, Benzyloxy, Phenoxy steht,
   gegebenenfalls in Gegenwart eines Säureakzeptors und gegebenenfalls in Gegenwart eines Verdünnungsmittels umsetzt,
   oder wenn man
(d) Triazolinone der allgemeinen Formel (II) 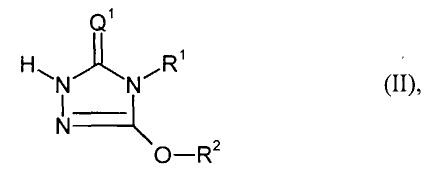 in welcher
   - Q¹, R¹ und R²: die oben angegebenen Bedeutungen haben,
   mit Sulfonsäurehalogeniden der allgemeinen Formel (VII)

   R³-SO₂-X (VII)

   in welcher
   - R³: die oben angegebene Bedeutung hat und
   - X: für Halogen steht,
   und Metall(thio)cyanaten der allgemeinen Formel (VIII)

   MQ²CN (VIII)

   in welcher
   - Q²: die oben angegebene Bedeutung hat und
   - M: für ein Alkalimetall oder Erdalkalimetall-äquivalent steht,
   gegebenenfalls in Gegenwart eines Reaktionshilfsmittels und gegebenenfalls in Gegenwart eines Verdünnungsmittels umsetzt,
   und gegebenenfalls die nach Verfahren (a), (b), (c) oder (d) erhaltenen Verbindungen der Formel (I) nach üblichen Methoden in Salze überführt.

Gegenstand der Erfindung sind bevorzugt Verbindungen der Formel (I), in welcher
- Q¹: für Sauerstoff steht,
- Q²: für Sauerstoff oder Schwefel steht,
- R¹: für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für Methoxy, Ethoxy, n- oder i-Propoxy oder für Allyloxy, für Methylamino, Ethylamino, n- oder i-Propylamino, n-, i-, s- oder t-Butylamino, Dimethylamino oder Diethylamino, für jeweils gegebenenfalls durch Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Fluor, Chlor, Brom, Cyano, Methyl, Trifluormethyl oder Methoxy substituiertes Benzyl oder Phenyl steht,
- R²: für Oxetanyl oder Tetrahydrofuryl steht und
- R³: für den Rest 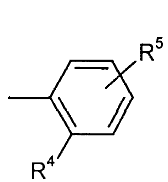 steht,
worin
R⁴ für Fluor, Chlor, Brom, Methyl, Ethyl, n- oder i-Propyl, Trifluormethyl, Methoxy, Ethoxy, n- oder i-Propoxy, n- oder i-Butoxy, Difluormethoxy, Trifluormethoxy, 2-Chlor-ethoxy, 2,2,2-Trifluor-ethoxy, 1,1,2,2-Tetrafluor-ethoxy, 1,1,2,2,2-Pentafluor-ethoxy, 2-Methoxyethoxy, Methylthio, Ethylthio, n- oder i-Propylthio, n- oder i-Butylthio, 2-Fluor-ethylthio, Allyloxy, Propargyloxy, Methylsulfinyl, Ethylsulfinyl, Methylsulfonyl, Ethylsulfonyl, Dimethylaminosulfonyl, Diethylaminosulfonyl, N-Methoxy-N-methylaminosulfonyl, Phenyl, Phenoxy, Methoxycarbonyl, Ethoxycarbonyl, n- oder i-Propoxycarbonyl steht und
R⁵ für Wasserstoff, Fluor, Chlor, Brom, Methyl, Ethyl, n- oder i-Propyl, Trifluorrnethyl, Methoxy, Ethoxy, n- oder i-Propoxy, Difluormethoxy, Trifluormethoxy, Methylthio oder Ethylthio steht;
weiterhin
- R³: für den Rest 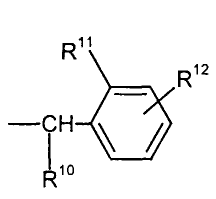 steht, worin
R¹⁰ für Wasserstoff steht,
R¹¹ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
R¹² für Wasserstoff steht; oder
- R³: für den Rest 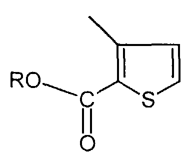 steht,
worin
R für Methyl, Ethyl, n- oder i-Propyl steht.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte. Diese Restedefinitionen können untereinander, also auch zwischen den angegebenen Bereichen bevorzugter Verbindungen, beliebig kombiniert werden.

Die bei den Restedefinitionen genannten Kohlenwasserstoffreste, wie Alkyl, Alkenyl oder Alkinyl, auch in Kombinationen mit Heteroatomen, wie in Alkoxy, Alkylthio oder Alkylamino, sind auch dann, wenn dies nicht ausdrücklich angegeben ist, geradkettig oder verzweigt.

Halogen steht im allgemeinen für Fluor, Chlor, Brom oder Iod, vorzugsweise für Fluor, Chlor oder Brom, insbesondere für Fluor oder Chlor.

Verwendet man beispielsweise 2-Trifluormethoxy-phenylsulfonyl-isocyanat und 4-Phenyl-5-(oxetan-3-yl-oxy)-2,4-dihydro-3H-1,2,4-triazol-3-on als Ausgangsstoffe, so kann der Reaktionsablauf beim erfindungsgemäßen Verfahren (a) durch das folgende Formelschema skizziert werden:

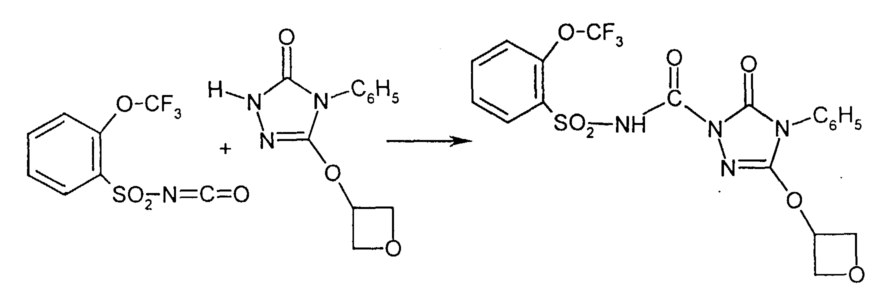

Die bei den erfindungsgemäßen Verfahren (a), (c) und (d) zur Herstellung von Verbindungen der Formel (I) als Ausgangsstoffe zu verwendenden Triazolinone sind durch die Formel (II) allgemein definiert.

In der Formel (II) haben Q¹, R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise für Q¹, R¹ und R² angegeben wurden.

Die Triazolinone der allgemeinen Formel (II) sind noch nicht aus der Literatur bekannt; sie sind als neue Stoffe auch Gegenstand der vorliegenden Anmeldung.

Man erhält die neuen Triazolinone der Formel (II), wenn man Carbazinsäureester der allgemeinen Formel (IX) 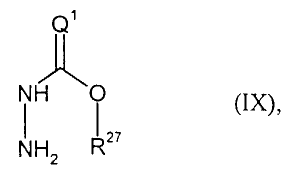 in welcher
- Q¹: die oben angegebene Bedeutung hat und
- R²⁷: für gegebenenfalls substituiertes Alkyl (vorzugsweise Methyl, Ethyl, Methoxyethyl oder Ethoxyethyl) oder für Phenyl steht,
mit Alkyliminokohlensäure-diestern der allgemeinen Formel (X) 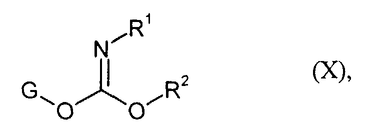 in welcher
- R¹ und R²: die oben angegebenen Bedeutungen haben und
- G: für Alkyl (vorzugsweise Methyl oder Ethyl) steht oder die gleiche Bedeutung wie R² hat,
gegebenenfalls in Gegenwart eines Verdünnungsmittels, wie z.B. Methanol, und gegebenenfalls in Gegenwart eines Reaktionshilfsmittels, wie z.B. Pivalinsäure, bei Temperaturen zwischen 0°C und 100°C umsetzt, die hierbei gebildeten Verbindungen der allgemeinen Formel (XI) 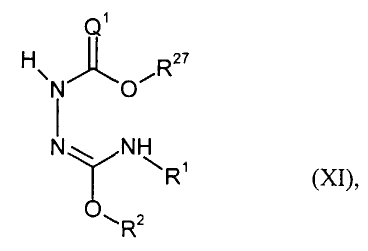 in welcher
- Q¹, R¹, R² und R²⁷: die oben angegebenen Bedeutungen haben,
- gegebenenfalls nach Zwischenisolierung - gegebenenfalls in Gegenwart eines Verdünnungsmittels, wie z.B. Methanol, und gegebenenfalls in Gegenwart eines Reaktionshilfsmittels, wie z.B. Natriummethylat, bei Temperaturen zwischen 20°C und 150°C cyclisierend kondensiert (vgl. die Herstellungsbeispiele).

Die beim erfindungsgemäßen Verfahren (a) zur Herstellung von Verbindungen der Formel (I) weiter als Ausgangsstoffe zu verwendenden Sulfonyliso(thio)cyanate sind durch die Formel (III) allgemein definiert.

In der Formel (III) haben Q² und R³ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q² und R³ angegeben wurden.

Die Ausgangsstoffe der Formel (III) sind bekannt und/oder können nach an sich bekannten Verfahren hergestellt werden (vgl. US-P 4127405, US-P 4169719, US-P 4371391, EP-A 7687, EP-A 13480, EP-A 21641, EP-A 23141, EP-A 23422, EP-A 30139, EP-A 35893, EP-A 44808, EP-A 44809, EP-A 48143, EP-A 51466, EP-A 64322, EP-A 70041, EP-A 173312).

Die beim erfindungsgemäßen Verfahren (b) zur Herstellung der Verbindungen der allgemeinen Formel (I) als Ausgangsstoffe zu verwendenden Triazolinon-Derivate sind durch die Formel (IV) allgemein definiert. In der Formel (IV) haben Q¹, Q², R¹ und R² vorzugsweise bzw. insbesondere diejenigen Bedeutungen, die bereits oben im Zusammenhang mit der Beschreibung der Verbindungen der Formel (I) als bevorzugt für Q¹, Q², R¹ und R² angegeben wurden; Z steht vorzugsweise für Fluor, Chlor, Brom, Methoxy, Ethoxy, Benzyloxy, Phenoxy, Halogen- oder Nitro-phenoxy, insbesondere für Methoxy, Phenoxy oder 4-Nitro-phenoxy.

Die Ausgangsstoffe der Formel (IV) sind noch nicht aus der Literatur bekannt; sie sind als neue Stoffe ebenfalls Gegenstand der vorliegenden Anmeldung.

Man erhält die neuen Verbindungen der Formel (IV), wenn man Triazolinone der allgemeinen Formel (II) 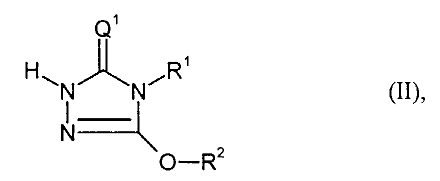 in welcher
- Q¹, R¹ und R²: die oben angegebenen Bedeutungen haben,
mit Kohlensäurederivaten der allgemeinen Formel (XII)

Z-CQ²-Z¹ (XII)

in welcher
- Q² und Z: die oben angegebenen Bedeutungen haben und
- Z¹: für Halogen (insbesondere Chlor), Alkoxy (insbesondere Methoxy), Aralkoxy (insbesondere Benzyloxy) oder Aryloxy (insbesondere Phenoxy) steht,
gegebenenfalls in Gegenwart eines Säureakzeptors, wie z.B. Natrium- oder Kaliumhydrid, Natrium- oder Kaliumhydroxid, Natrium- oder Kalium-t-butylat, und gegebenenfalls in Gegenwart eines Verdünnungsmittels, wie z.B. Tetrahydrofuran oder Dimethoxyethan, oder in einem Zweiphasensystem aus Wasser und einem organischen Lösungsmittel, wie z.B. Methylenchlorid oder Chloroform, bei Temperaturen zwischen 0°C und 100°C umsetzt.

Die beim erfindungsgemäßen Verfahren (b) zur Herstellung der Verbindungen der allgemeinen Formel (I) weiter als Ausgangsstoffe zu verwendenden Sulfonsäureamide sind durch die Formel (V) allgemein definiert. In der Formel (V) hat R³ vorzugsweise bzw. insbesondere diejenige Bedeutung, die bereits oben im Zusammenhang mit der Beschreibung der Verbindungen der Formel (I) als bevorzugt bzw. als insbesondere bevorzugt für R³ angegeben wurde.

Die Ausgangsstoffe der Formel (V) sind bekannt und/oder können nach an sich bekannten Verfahren hergestellt werden (vgl. US-P 4127405, US-P 4169719, US-P 4371391, EP-A 7687, EP-A 13480, EP-A 21641, EP-A 23141, EP-A 23422, EP-A 30139, EP-A 35893, EP-A 44808, EP-A 44809, EP-A 48143, EP-A 51466, EP-A 64322, EP-A 70041, EP-A 173312).

Die beim erfindungsgemäßen Verfahren (c) zur Herstellung der Verbindungen der Formel (I) als Ausgangsstoffe zu verwendenden Sulfonsäureamid-Derivate sind durch die Formel (VI) allgemein definiert. In der Formel (VI) haben Q² und R³ vorzugsweise bzw. insbesondere diejenigen Bedeutungen, die bereits oben im Zusammenhang mit der Beschreibung der Verbindungen der Formel (I) als bevorzugt für Q² und R³ angegeben wurde; Z steht vorzugsweise für Fluor, Chlor, Brom, Methoxy, Ethoxy, Benzyloxy oder Phenoxy, insbesondere für Methoxy oder Phenoxy.

Die Ausgangsstoffe der Formel (VI) sind bekannt und/oder können nach an sich bekannten Verfahren hergestellt werden.

Die beim erfindungsgemäßen Verfahren (d) zur Herstellung der Verbindungen der Formel (I) als Ausgangsstoffe zu verwendenden Sulfonsäurehalogenide sind durch die Formel (VII) allgemein definiert. In der Formel (VII) hat R³ vorzugsweise bzw. insbesondere diejenige Bedeutung, die bereits oben im Zusammenhang mit der Beschreibung der Verbindungen der Formel (I) als bevorzugt für R³ angegeben wurde; X steht vorzugsweise für Fluor, Chlor oder Brom, insbesondere für Chlor.

Die Ausgangsstoffe der Formel (VII) sind bekannt und/oder können nach an sich bekannten Verfahren hergestellt werden.

Die erfindungsgemäßen Verfahren (a), (b), (c) und (d) zur Herstellung der neuen Verbindungen der Formel (I) werden vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol; Ether wie Diethyl- und Dibutylether, Glykoldimethylether und Diglykoldimethylether, Tetrahydrofuran und Dioxan; Ketone wie Aceton, Methyl-ethyl-, Methyl-isopropyl- und Methyl-isobutyl-keton; Ester wie Essigssäuremethylester und -ethylester; Nitrile wie z.B. Acetonitril und Propionitril; Amide wie z.B. Dimethylformamid, Dimethylacetamid und N-Methylpyrrolidon, sowie Dimethylsulfoxid, Tetramethylensulfon und Hexamethylphosphorsäuretriamid.

Als Reaktionshilfsmittel bzw. als Säureakzeptoren können bei den erfindungsgemäßen Verfahren (a), (b), (c) und (d) alle üblicherweise für derartige Umsetzungen verwendbaren Säurebindemittel eingesetzt werden. Vorzugsweise in Frage kommen Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kalium-tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäßen Verfahren (a), (b), (c) und (d) in einem größeren Bereich variiert werden. Im allgemeinen arbeitet man bei Temperaturen zwischen -20°C und +100°C, vorzugsweise bei Temperaturen zwischen 0°C und +80°C.

Die erfindungsgemäßen Verfahren (a), (b), (c) und (d) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b), (c) und (d) werden die jeweils benötigten Ausgangsstoffe im allgemeinen in angenähert äquimolaren Mengen eingesetzt. Es ist jedoch auch möglich, eine der jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b), (c) und (d) jeweils nach üblichen Methoden (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B Erdölfraktionen, mineralische und pflanzliche Öle, Alkohole, wie Butanol oder Glykol sowie deren Ether und Ester, Ketone wie Aceton, Methyl-ethylketon, Methyl-isobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfopmethyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 2 kg pro ha.

Die Herstellung und die Verwendung der erfindungsgemäßen Wirkstoffe geht aus den nachfolgenden Beispielen hervor.

### Herstellungsbeispiele:

### Beispiel 1

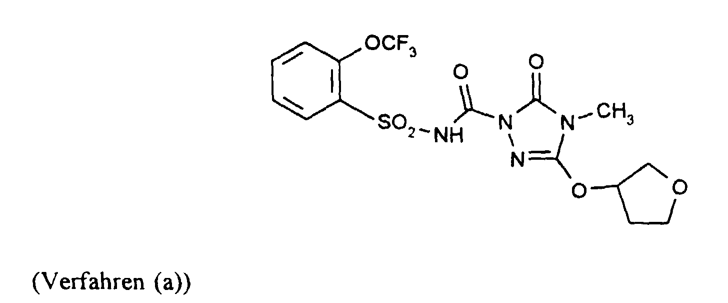

Eine Mischung aus 1,3 g (7 mMol) 4-Methyl-5-(3-tetrahydrofuryl-oxy)-2,4-dihydro-3H-1,2,4-triazol-3-on, 2,24 g (8,4 mMol) 2-Trifluormethoxy-phenylsulfonylisocyanat und 60 ml Acetonitril wird 16 Stunden bei ca. 20°C gerührt. Dann wird im Wasserstrahlvakuum eingeengt, der Rückstand mit Isopropanol/Petrolether (Vol. ca. 3/7) verrührt und das hierbei kristallin angefallene Produkt durch Absaugen isoliert.

Man erhält 3,0 g (94% der Theorie) 4-Methyl-5-(3-tetrahydrofuryl-oxy)-2-(2-trifluormethoxy-phenylsulfonylaminocarbonyl)-2,4-dihydro-3H-1,2,4-triazol-3-on vom Schmelzpunkt 184°C.

### Beispiel 2

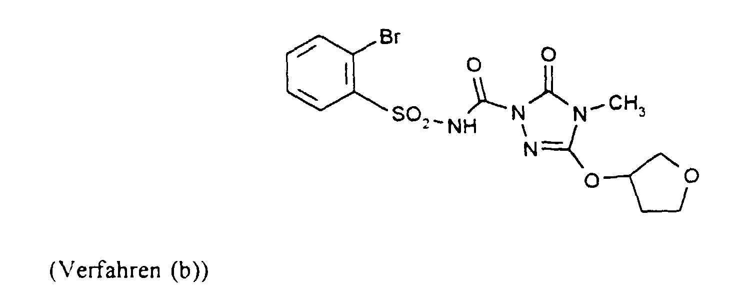

2,5 g (10 mMol) 2-Brom-benzolsulfonamid werden in 40 ml Acetonitril gelöst und unter Rühren mit 3,2 g (10 mMol) 4-Methyl-2-phenoxycarbonyl-5-(3-tetrahydrofuryl-oxy)-2,4-dihydro-3H-1,2,4-triazol-3-on und 1,6 g (10 mMol) Diazabicylo-undecen (DBU) versetzt. Die Mischung wird 12 Stunden bei 20°C gerührt und anschließend im Wasserstrahlvakuum eingeengt. Der Rückstand wird in Methylenchlorid aufgenommen, mit 10%iger Salzsäure gewaschen, mit Natriumsulfat getrocknet und filtriert. Das Filtrat wird im Wasserstrahlvakuum eingeengt, der Rückstand mit Isopropanol/Petrolether (1/5) digeriert und das kristallin angefallene Produkt durch Absaugen isoliert.

Man erhält 3,5 g (78% der Theorie) 2-(2-Brom-phenylsulfonyl-aminocarbonyl)-4-methyl-5-(3-tetrahydrofuryl-oxy)-2,4-dihydro-3H-1,2,4-triazol-3-on vom Schmelzpunkt 191°C.

Analog Beispiel 1 oder 2 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

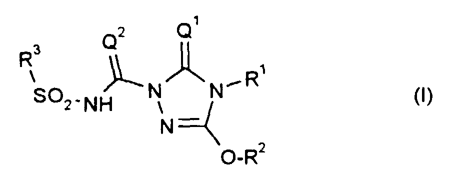

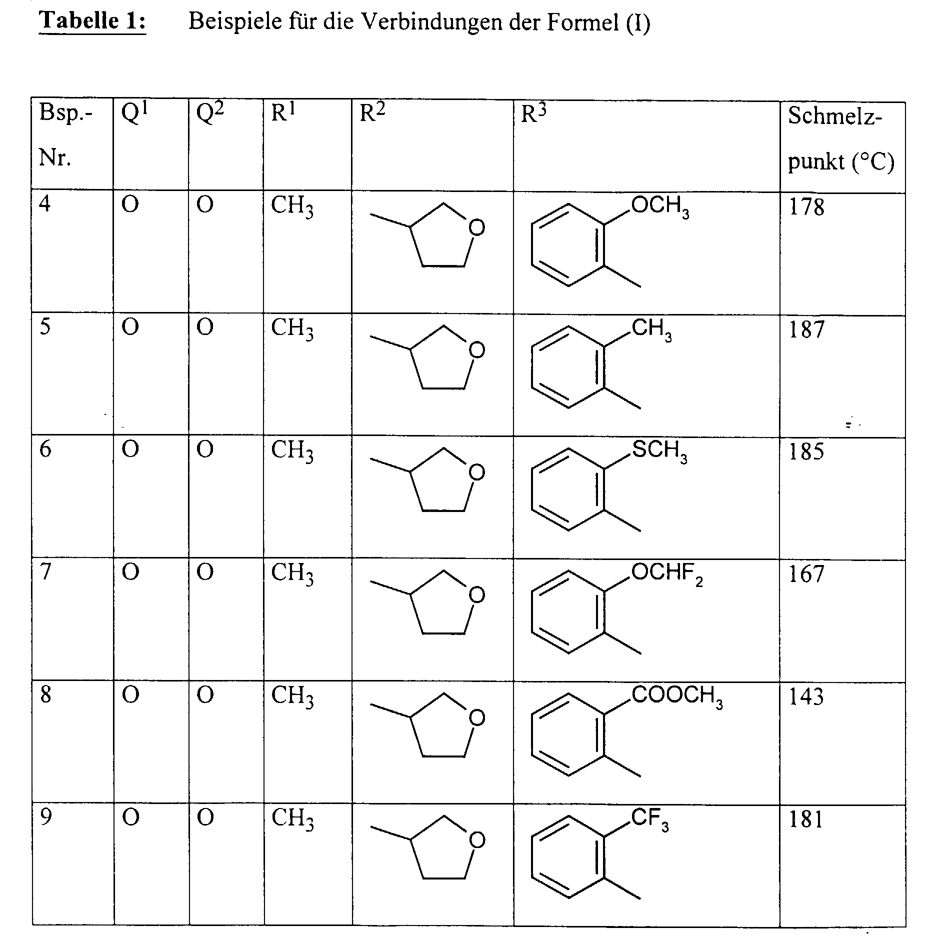

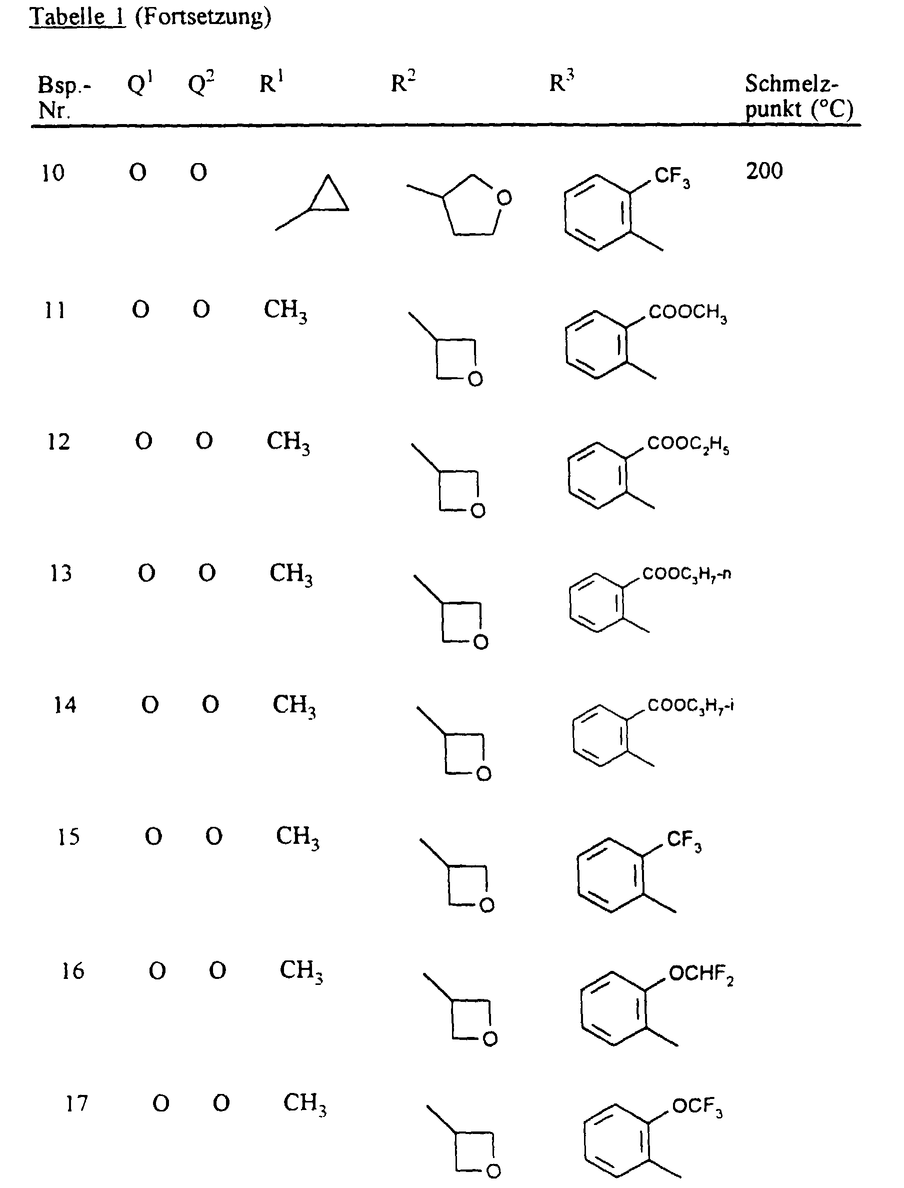

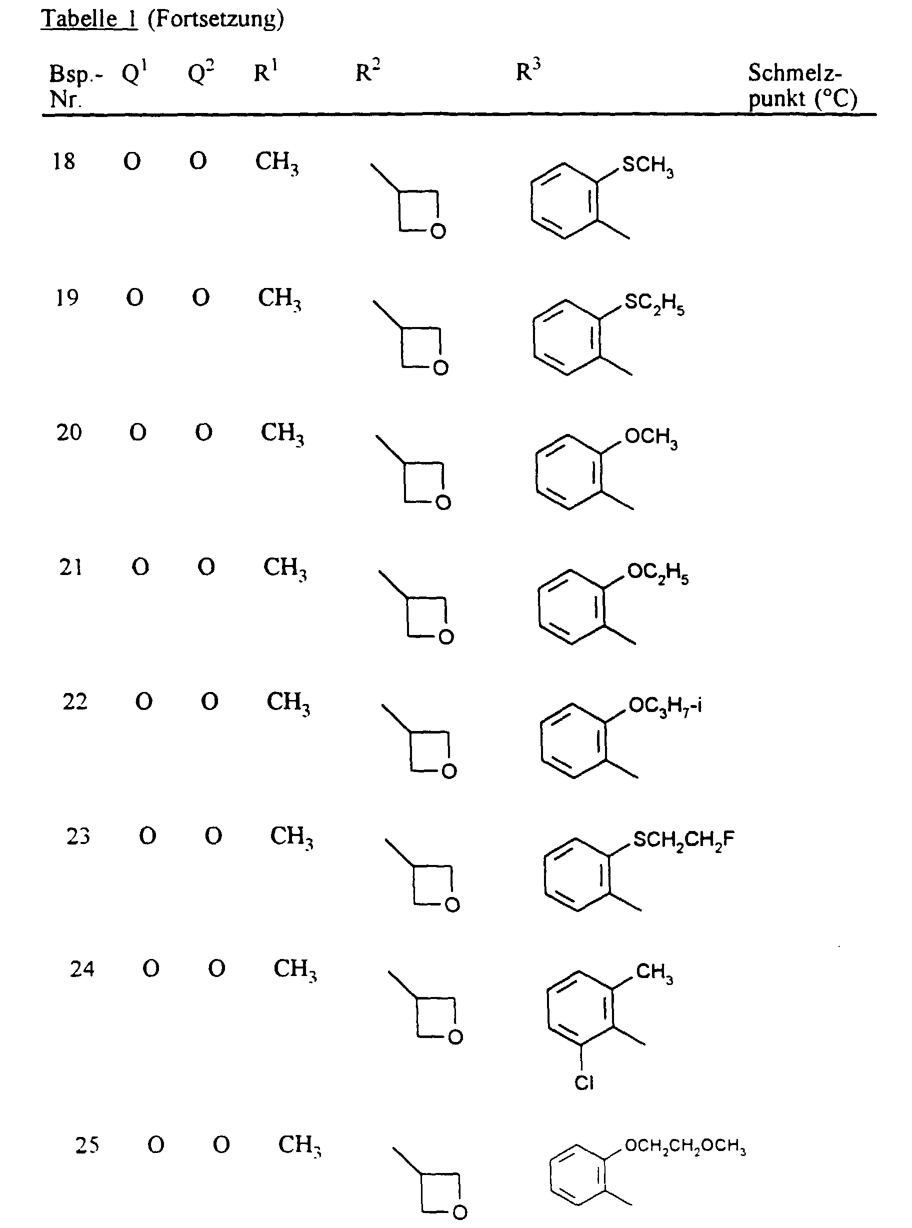

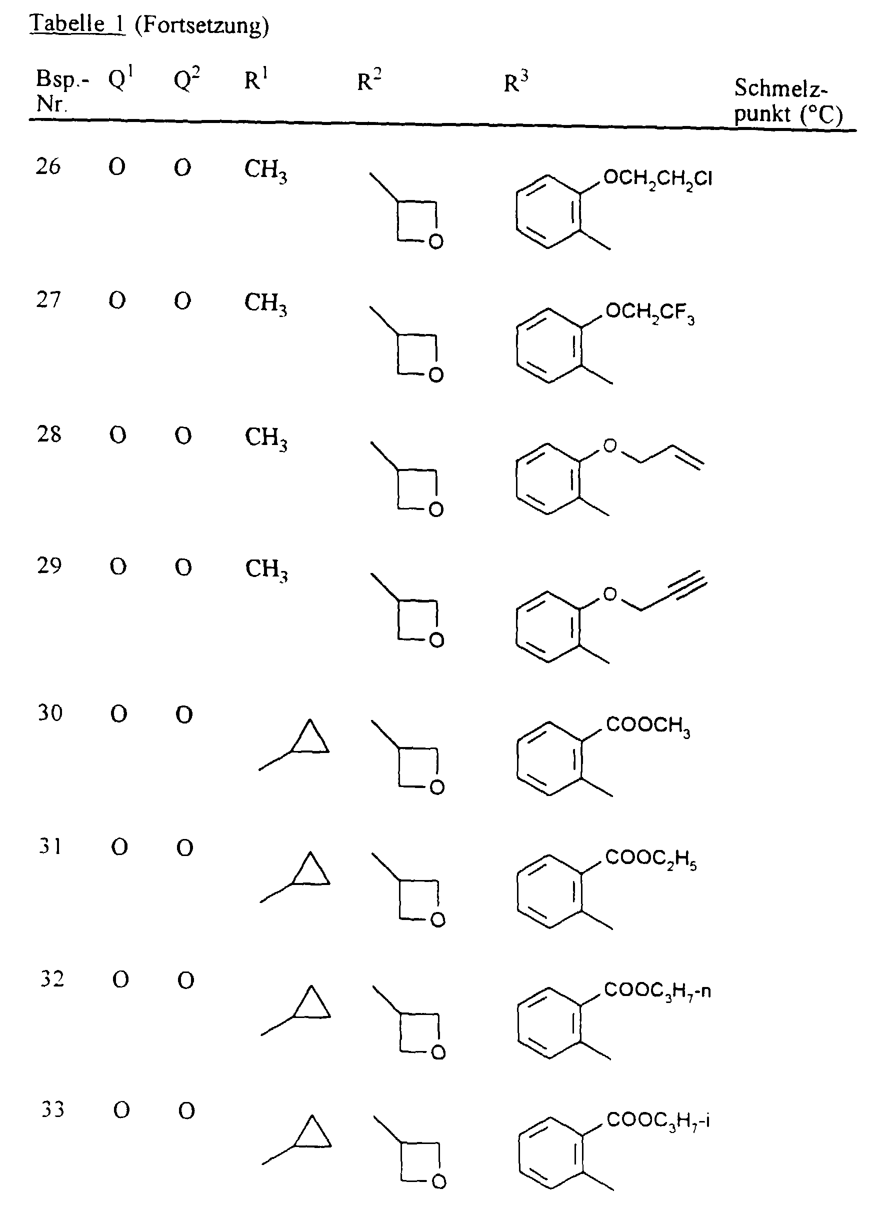

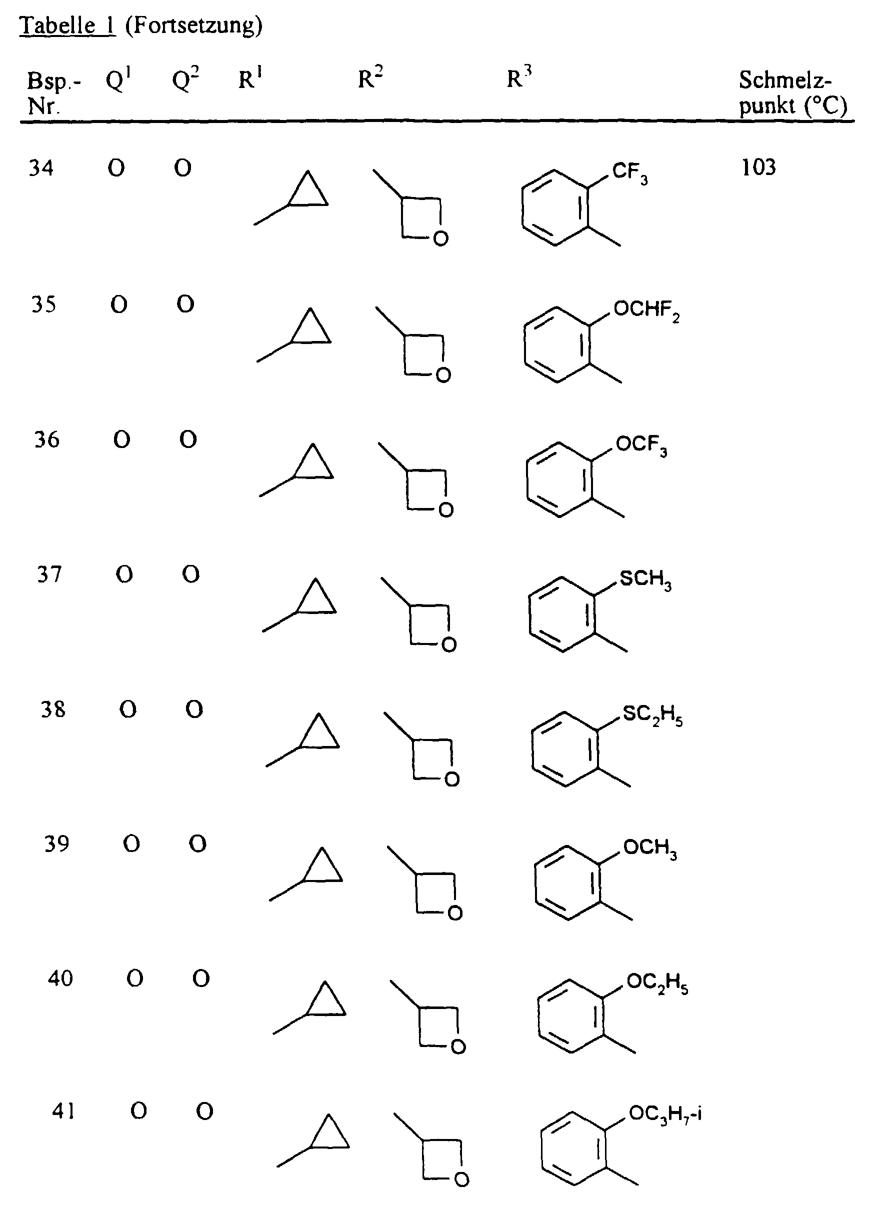

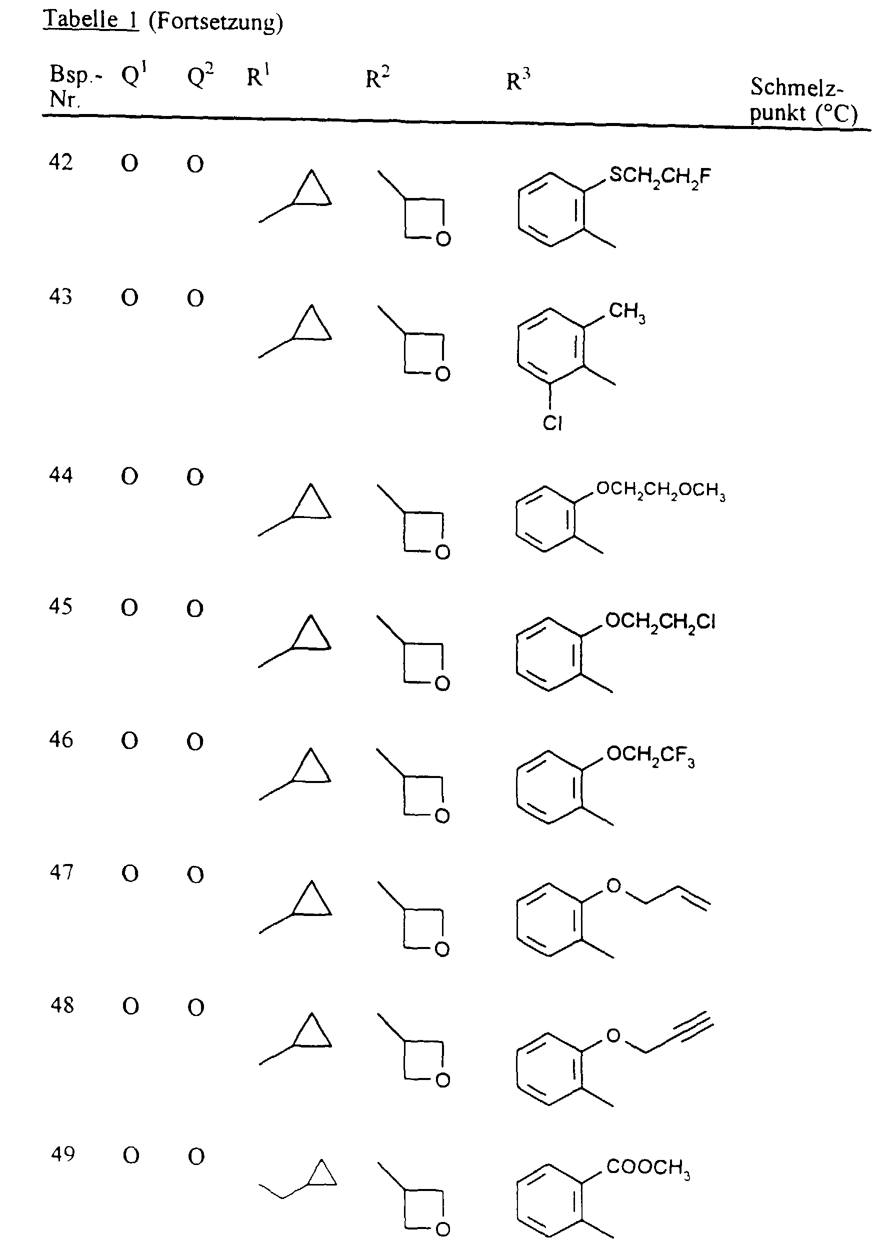

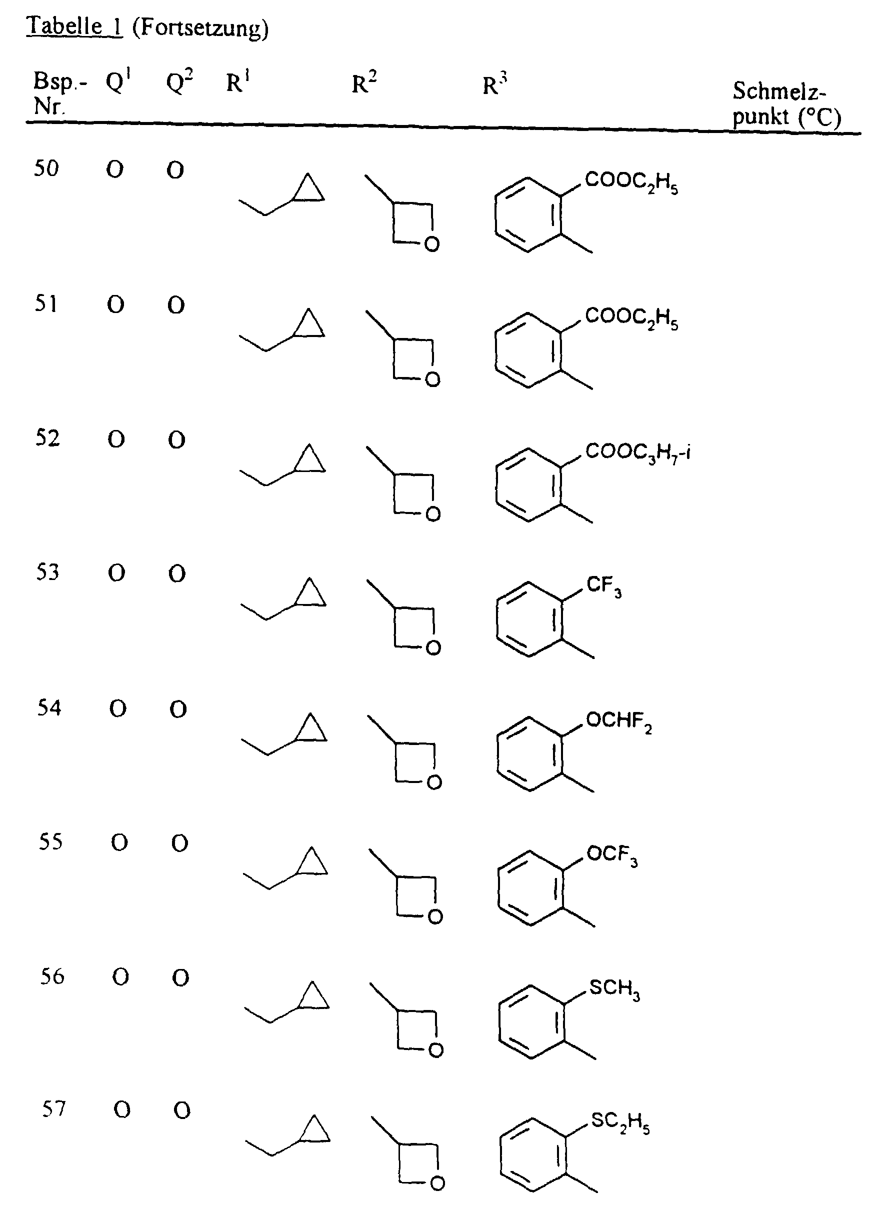

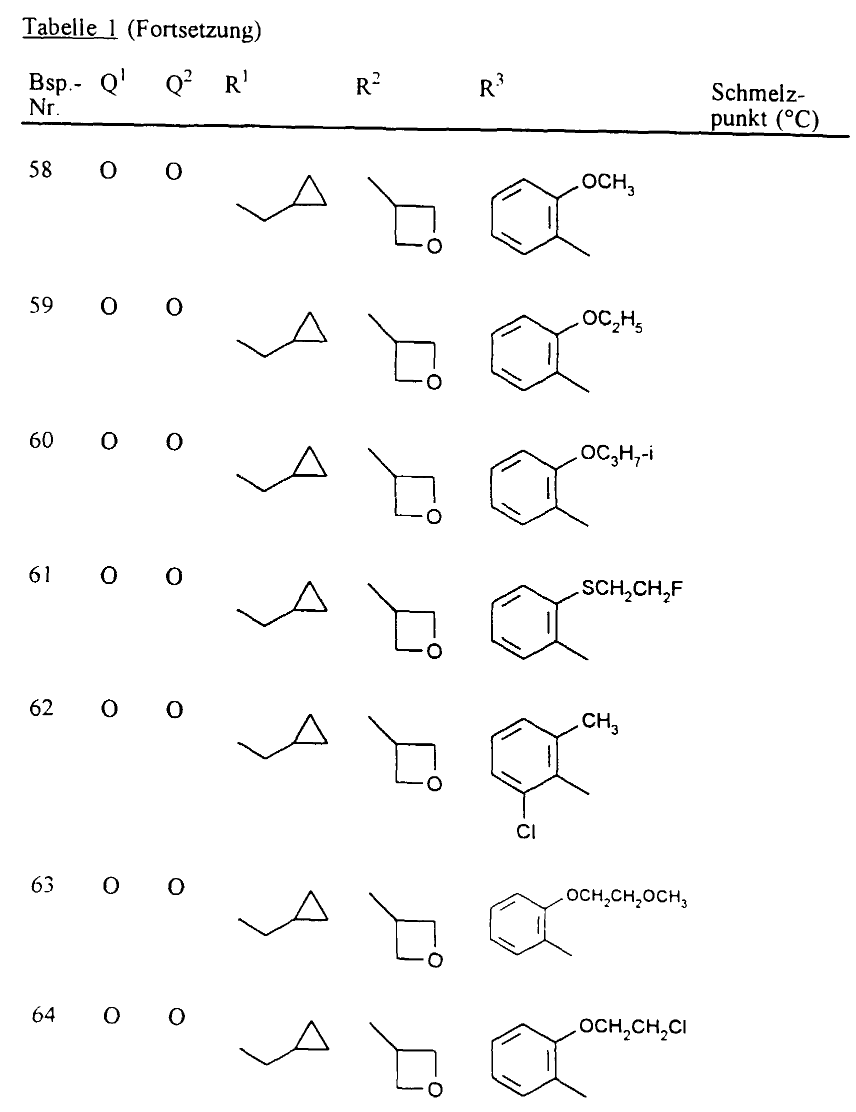

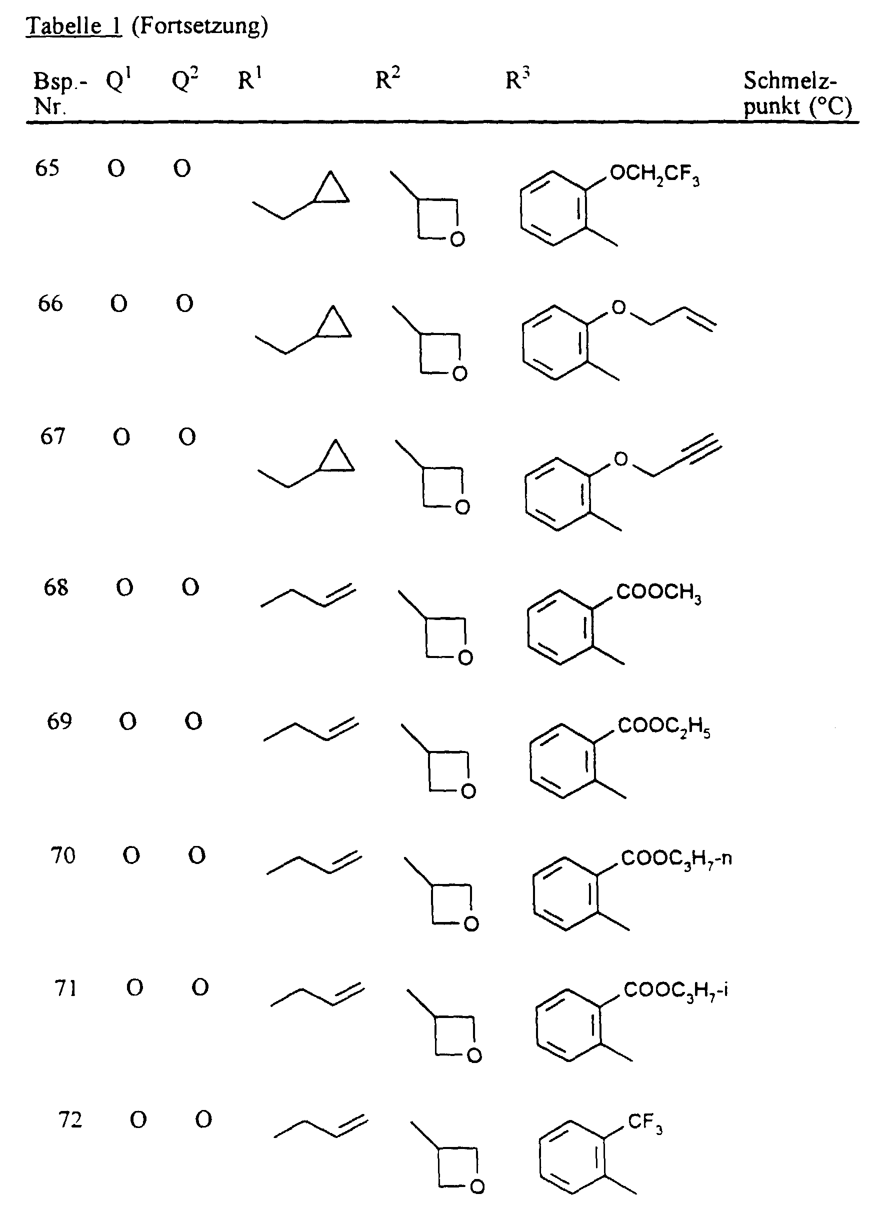

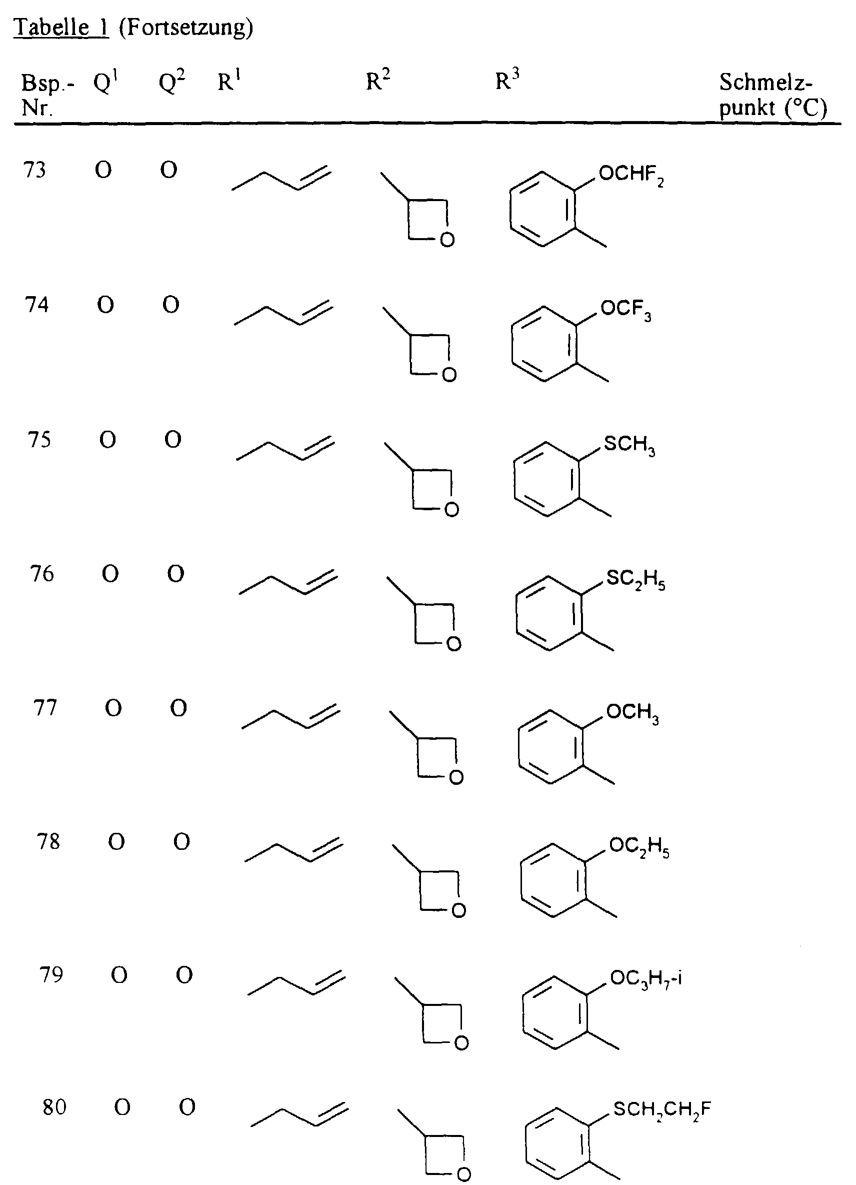

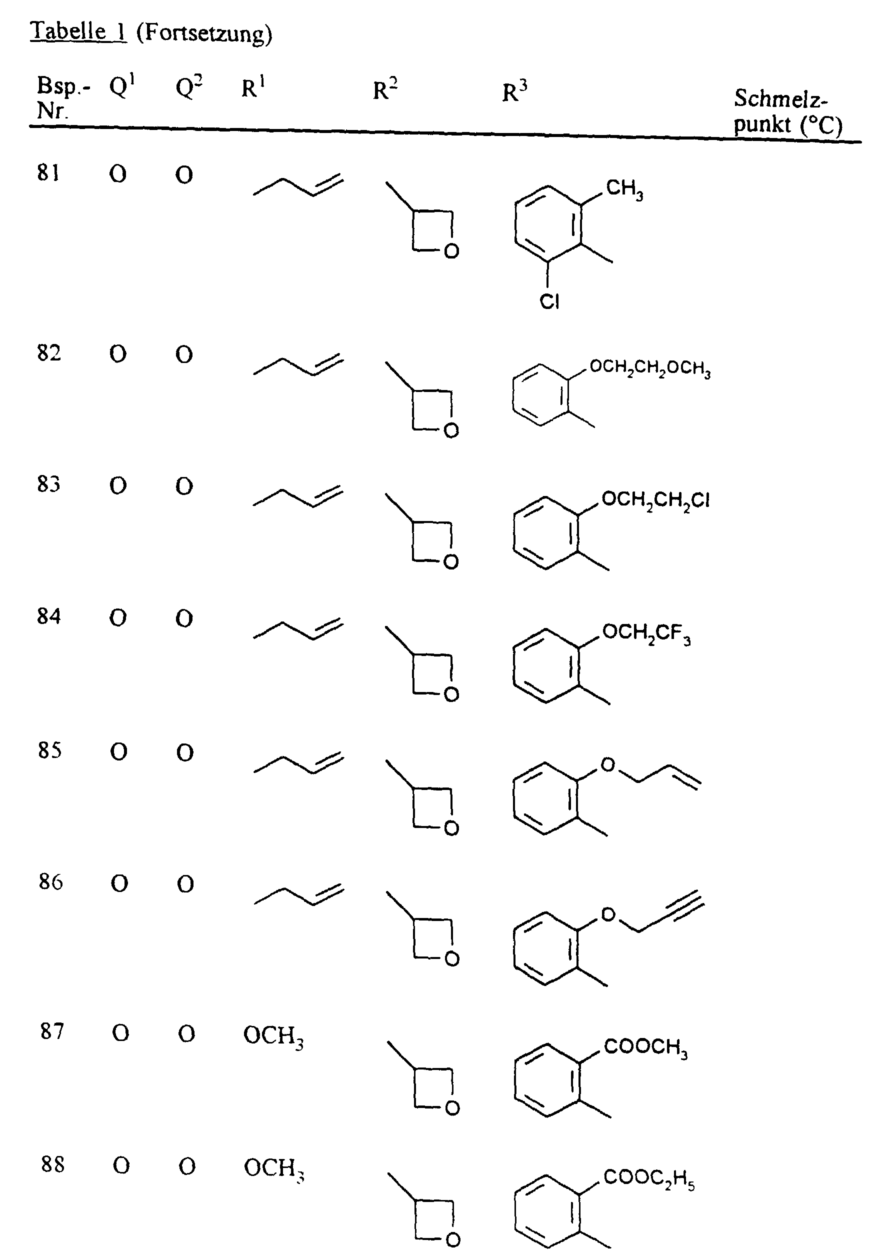

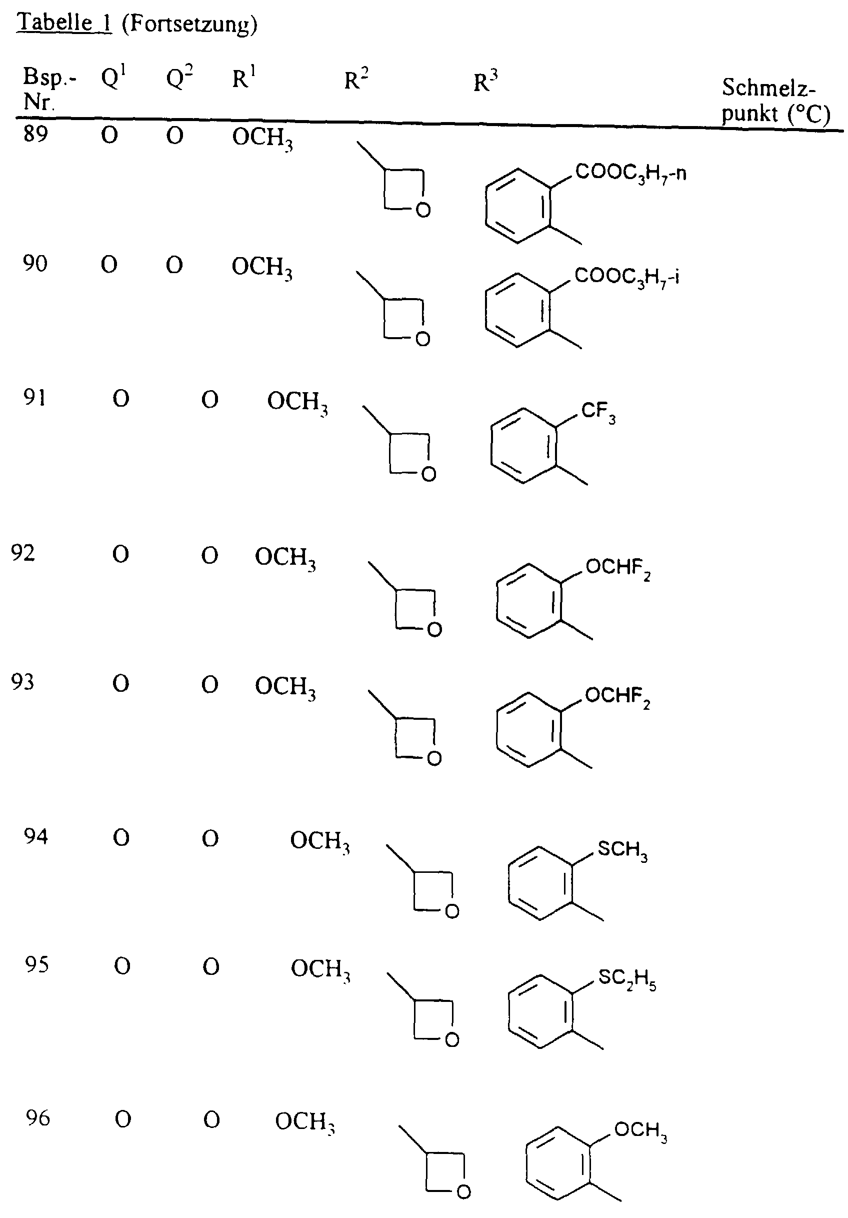

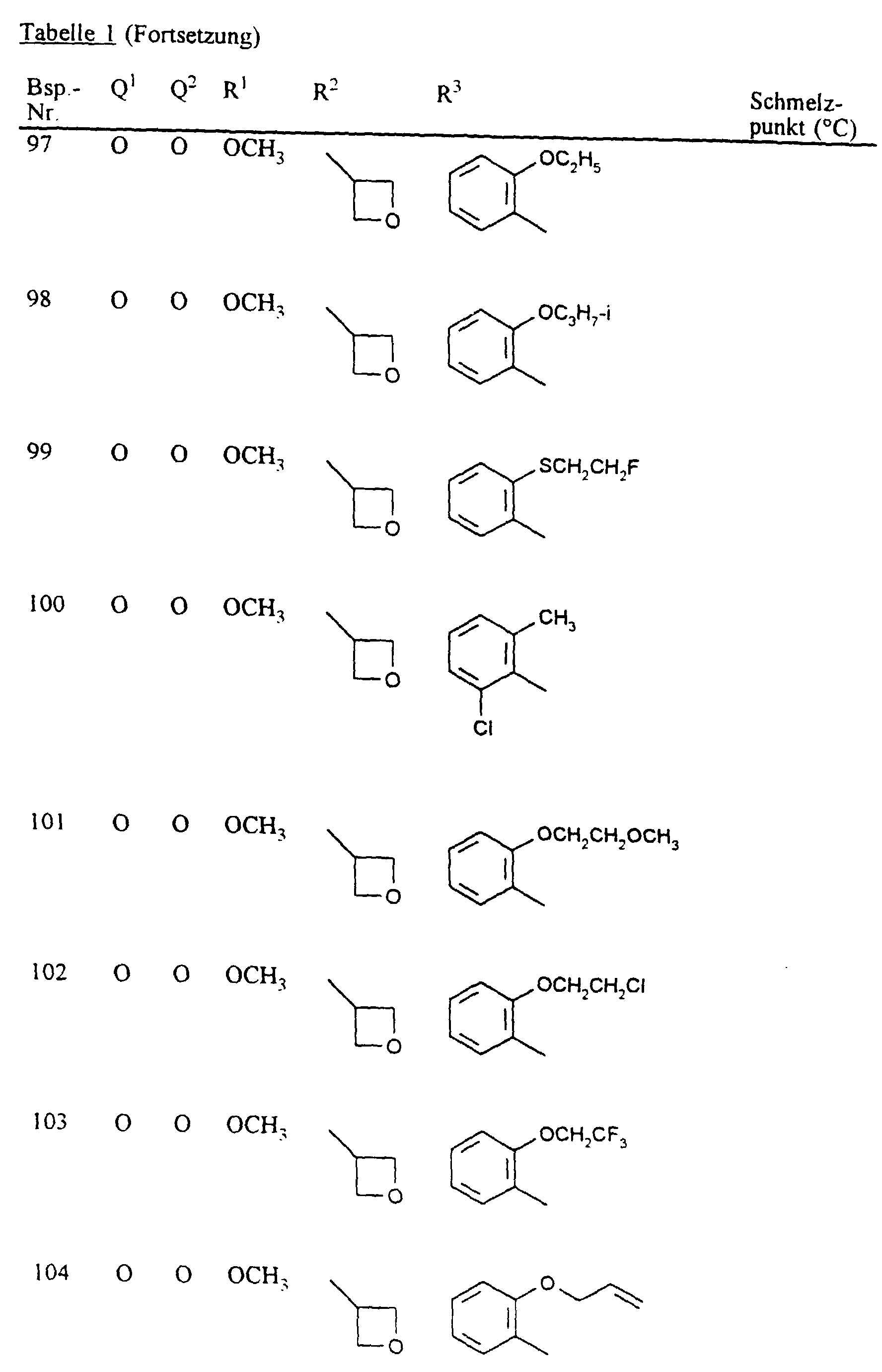

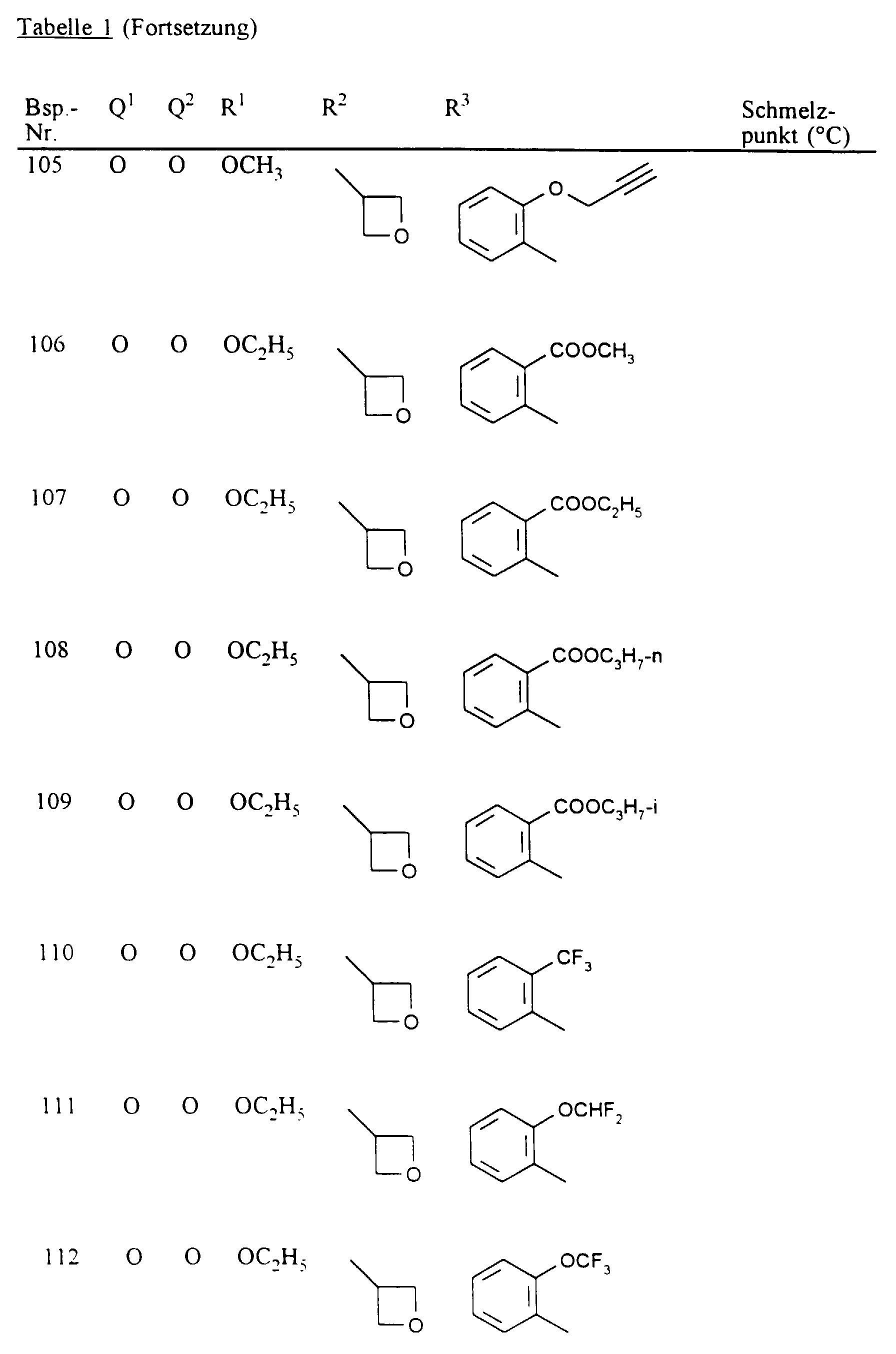

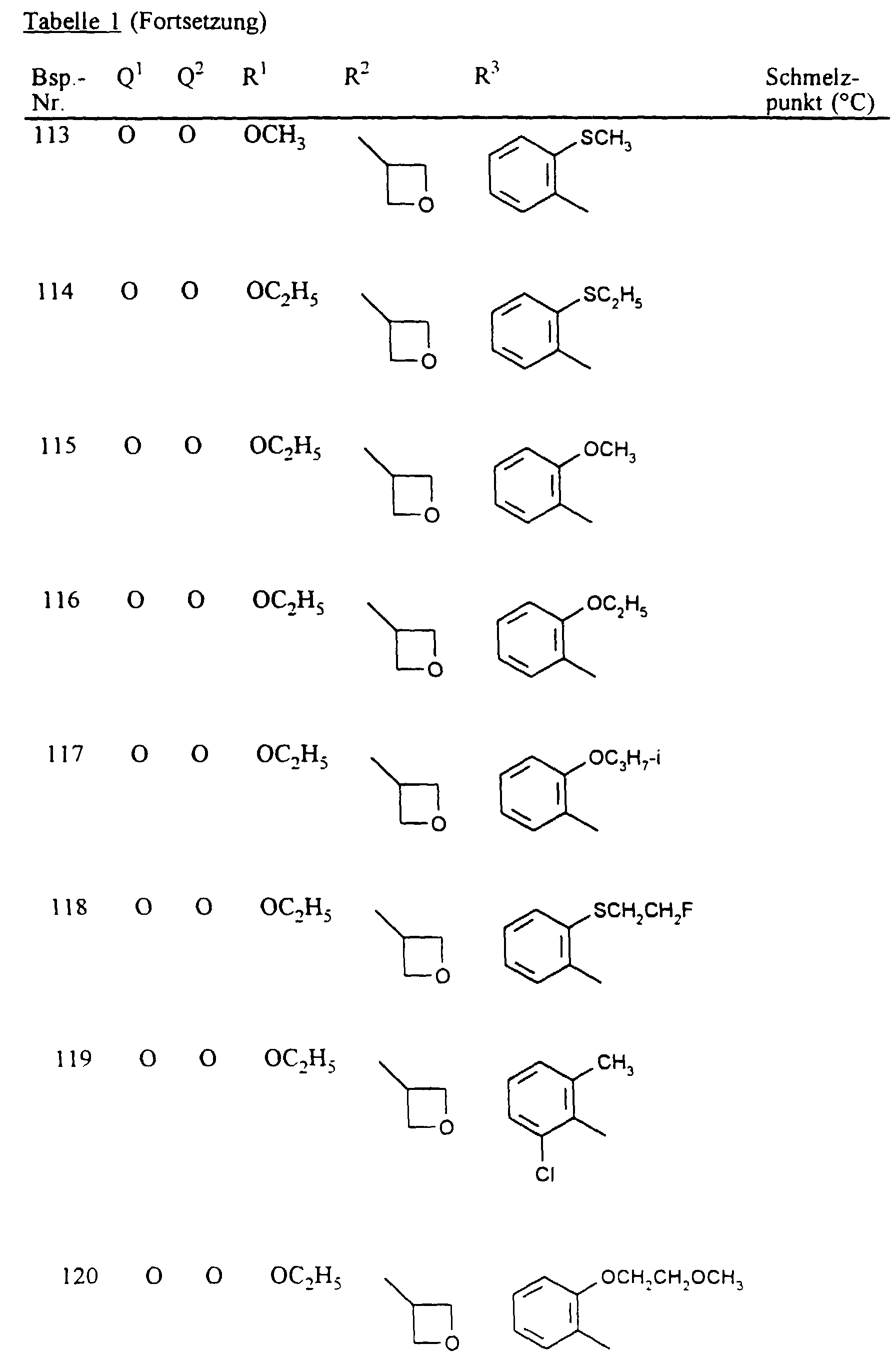

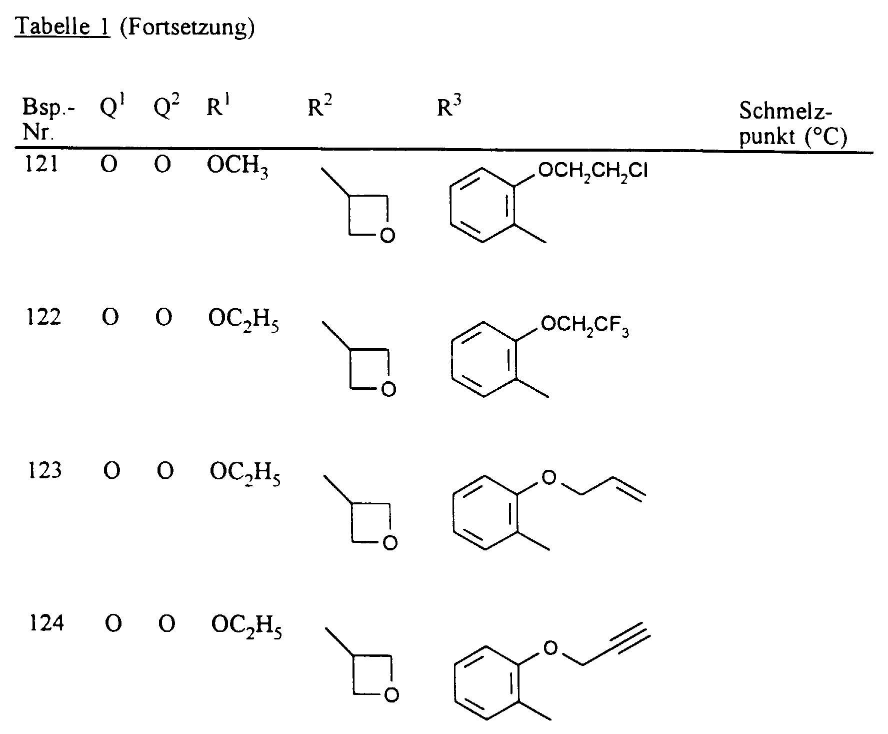

### Ausgangsstoffe der Formel (II):

### Beispiel (II-1)

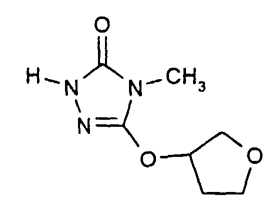

### Stufe 1:

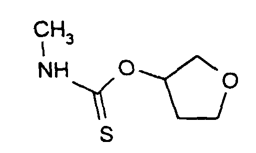

233 g (2,65 Mol) 3-Hydroxy-tetrahydrofuran werden in 750 ml Toluol bei 50 mbar im Wasserstrahlvakuum azeotrop entwässert. Dann werden unter Eiskühlung innerhalb von ca. 20 Minuten 18 g (0,60 Mol) Natriumhydrid (80%ig) eingetragen. Nach 30 Minuten bei 50°C werden unter Eiskühlung 176 g (2,41 Mol) Methylisothiocyanat innerhalb von ca. 30 Minuten zugetropft. Dann wird die Reaktionsmischung noch ca. 2 Stunden bei 50°C gerührt. Anschließend wird unter Eiskühlung mit 71,2 g 30,7%iger Salzsäure (0,60 Mol HCl) neutralisiert, schließlich bei 100 mbar azeotrop entwässert, filtriert und das Filtrat auf ca. 500 g eingeengt.

### Stufe 2:

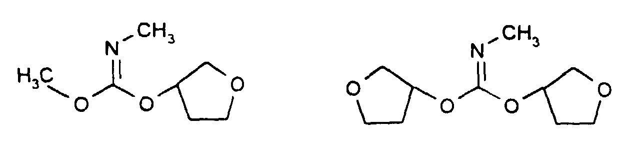

782 g (2,65 Mol) Dibutylzinn-dimethoxid werden bei 40°C im Ölpumpenvakuum vorgelegt und die bei Stufe 1 erhaltene Produktlösung wird innerhalb von ca 30 Minuten zugetropft. Dabei wird Methanol abdestilliert und die Temperatur auf ca. 80°C erhöht Anschließend wird die Luft durch Stickstoff ersetzt und die Reaktionsmischung 16 Stunden bei 100°C gerührt. Dann wird im Ölpumpenvakuum rektifiziert. Nach Abdestillieren von 43 g Vorlauf werden 290 ml Hauptlauf (bei 1 mbar/60°C) erhalten, die laut gaschromatographischer Analyse aus 203,5 g (1,28 Mol) Methylimino-kohlensäure-O-methylester-O-(3-tetrahydrofuryl)-ester und 75,2 g (0,35 Mol) Methylimino-kohlensäure-O,O-bis-(3-tetrahydrofuryl)-ester bestehen.

### Stufe 3:

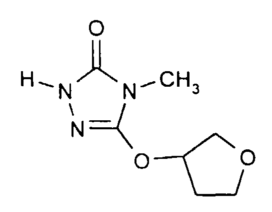

261 g (1,63 Mol) Carbazinsäure-(2-ethoxyethyl)-ester werden bei 0°C vorgelegt und das auf 0°C abgekühlte Produktgemisch aus Stufe 2 in einem Guß dazu gegeben. Dann werden in Abständen von ca. 20 Minuten jeweils 1,66 g Pivalinsäure zugesetzt und die Mischung wird 16 Stunden bei 20°C und dann noch 30 Minuten bei 45°C gerührt. Nach Zugabe von 299 g 30%iger methanolischer Natriummethylat-Lösung (1,66 Mol Natriummethylat) wird die Mischung noch 3 Stunden bei 55°C gerührt. Nach Erkalten wird unter Eiskühlung mit 197 g 30,7%iger Salzsäure (1,66 Mol HCl) neutralisiert und dann im Wasserstrahlvakuum eingeengt. Der Rückstand wird aus 750 ml Toluol umkristallisiert. Man erhält 209 g eines Produktgemisches, welches nach gaschromatographischer Analyse zu 51% aus 4-Methyl-5-methoxy-2,4-dihydro-3H-1,2,4-triazol-3-on und zu 43% aus 4-Methyl-5-(3-tetrahydrofuryl-oxy)-2,4-dihydro-3H-1,2,4-triazol-3-on besteht. Das Produktgemisch wird aus 400 ml Wasser umkristallisiert, wobei man 50 g 4-Methyl-5-methoxy-2,4-dihydro-3H-1,2,4-triazol-3-on als kristallines Produkt erhält. Die Mutterlauge wird eingedampft und der Rückstand aus 400 ml Toluol umkristallisiert. Nach erneuter Umkristallisation des Produktes aus Methanol erhält man 83 g (0,45 Mol) 4-Methyl-5-(3-tetrahydrofuryl-oxy)-2,4-dihydro-3H-1,2,4-triazol-3-on vom Schmelzpunkt 164°C.

Analog Beispiel (II-1) können beispielsweise auch die in der nachstehenden Tabelle 2 aufgeführten Verbindungen der Formel (II) hergestellt werden.

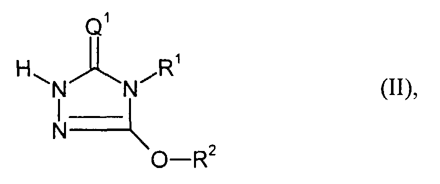

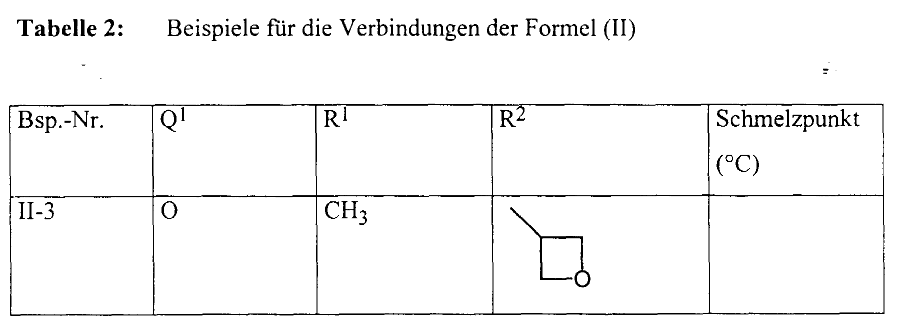

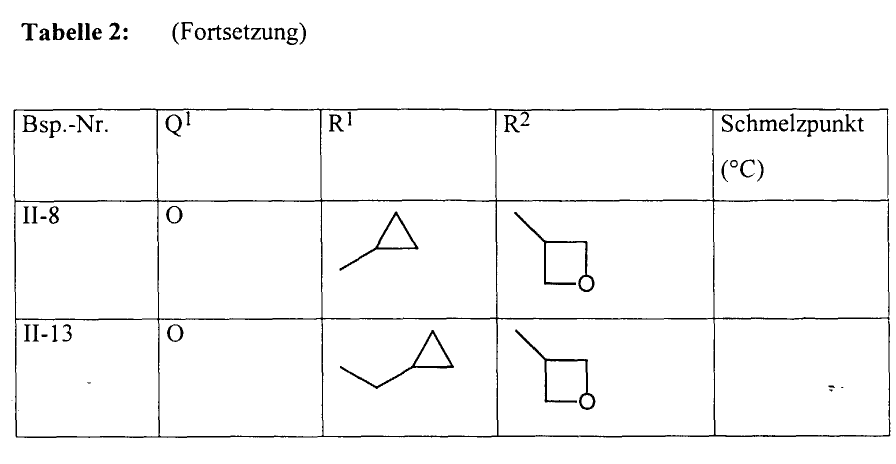

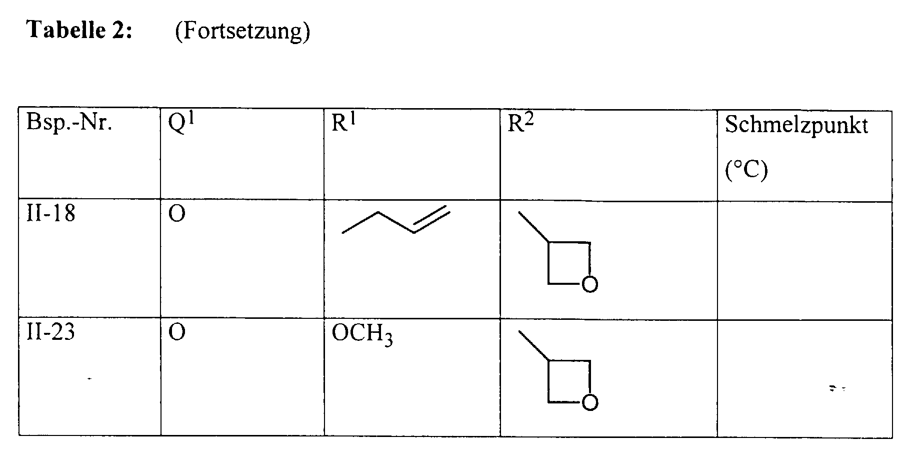

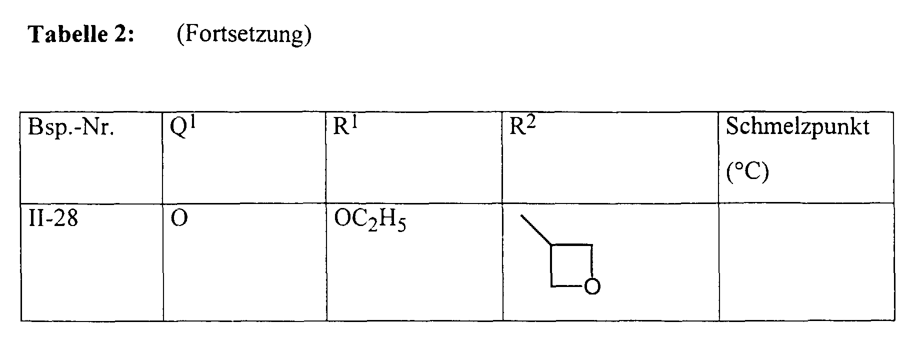

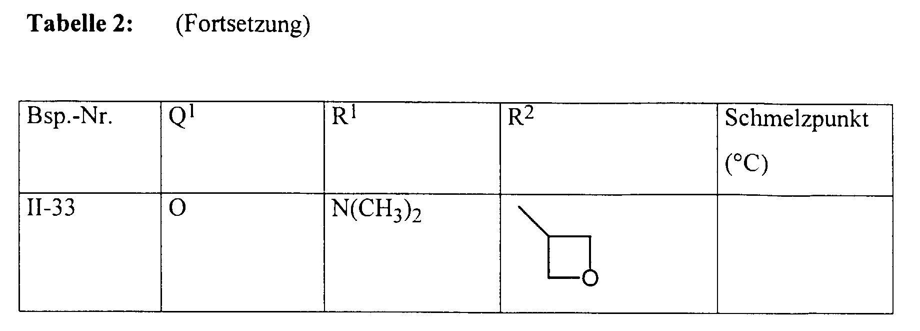

### Ausgangsstoffe der Formel (IV):

### Beispiel (IV-1)

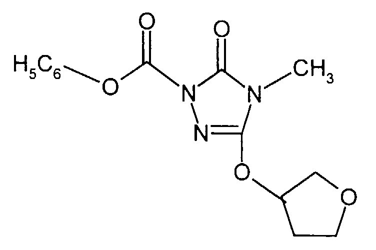

6,9 g (44 mmol) Chlorameisensäure-phenylester werden bei 20°C bie 30°C tropfenweise unter Rühren zu einer Mischung aus 7,4 g (40 mmol) 4-Methyl-5-(3-tetrahydrofuryloxy)-2,4-dihydro-3H-1,2,4-triazol-3-on, 1,8 g (44 mmol) Natriumhydroxid, 0,2 g Tetrabutylammoniumchlorid, 50 ml Wasser und 50 ml Methylenchlorid gegeben und die Reaktionsmischung wird 12 Stunden bei 20°C gerührt. Anschließend wird die organische Phase abgetrennt, mit Wasser gewaschen, mit Natriumsulfat getrocknet und filtriert. Das Filtrat wird im Wasserstrahlvakuum eingeengt, der Rückstand mit Petrolether digeriert und das hierbei kristallin erhaltene Produkt durch Absaugen isoliert.

Man erhält 11,8 g (97 % der Theorie) 4-Methyl-2-phenoxycarbonyl-5-(3-tetrahydrofuryloxy)-2,4-dihydro-3H-1,2,4-triazol-3-on vom Schmelzpunkt 98°C.

Analog Beispiel (IV-1) können beispielsweise auch die in der nachstehenden Tabelle 3 aufgeführten Verbindungen der Formel (IV) hergestellt werden.

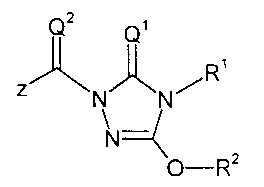

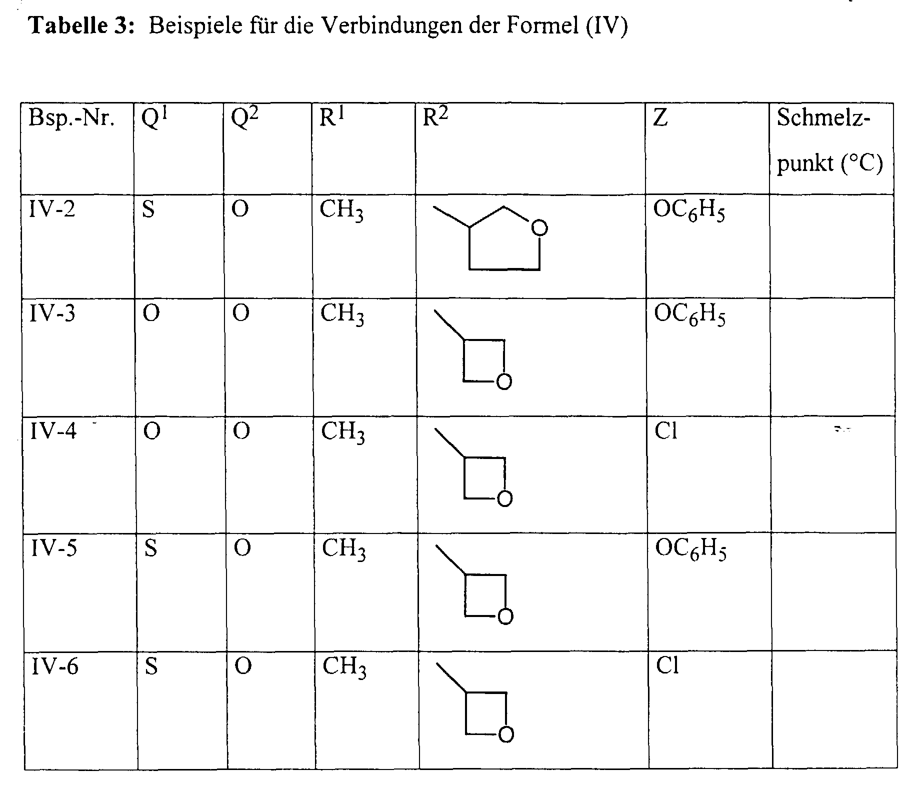

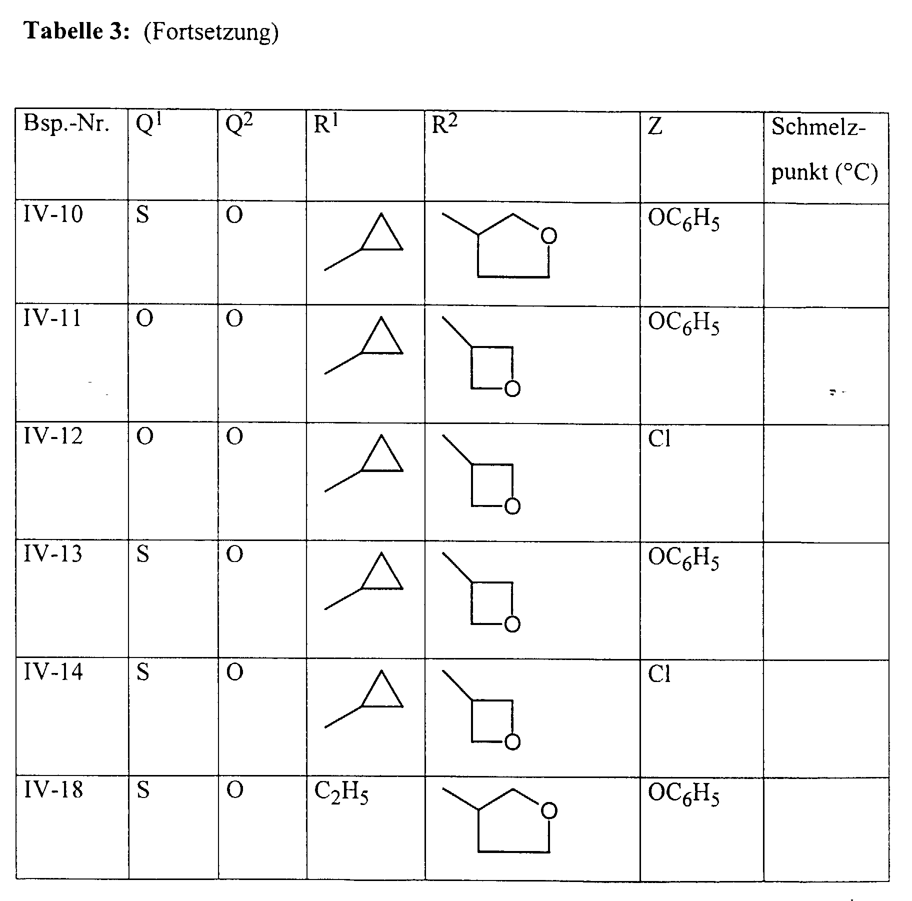

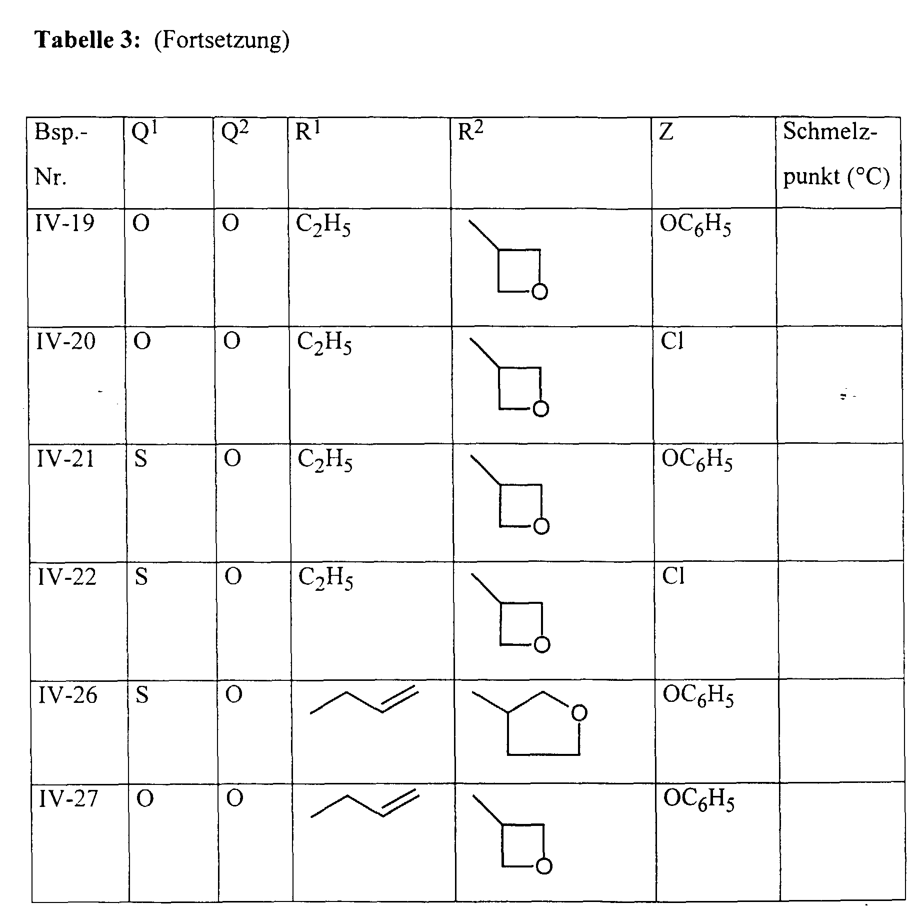

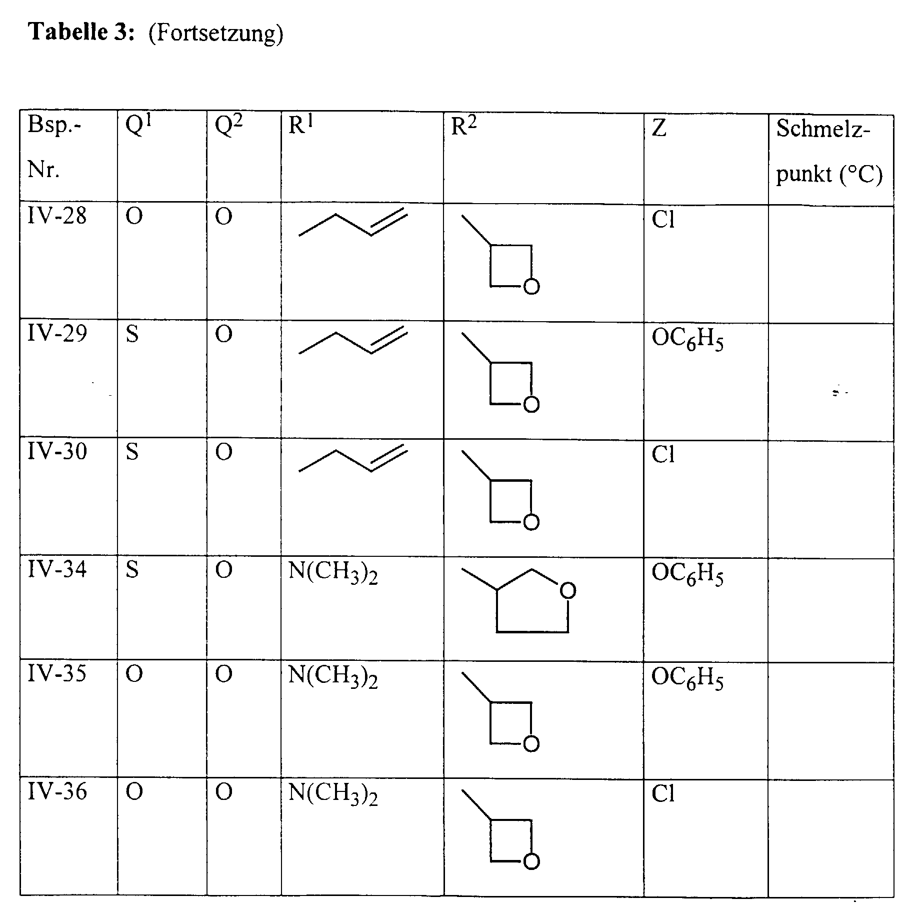

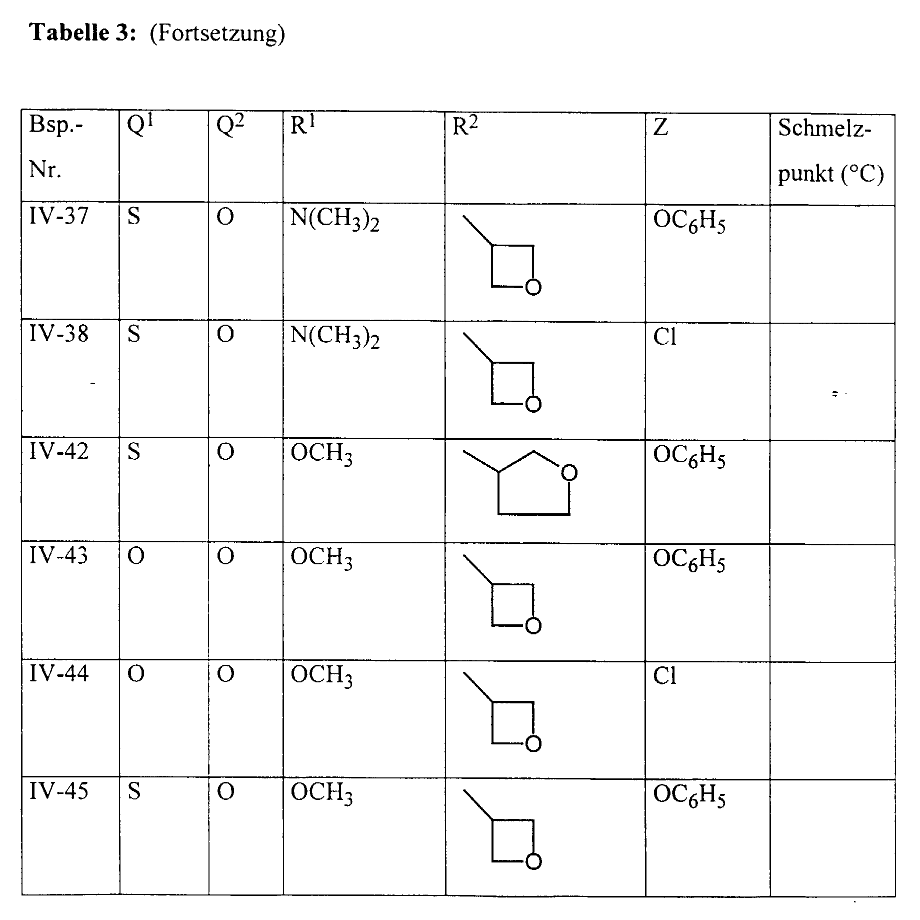

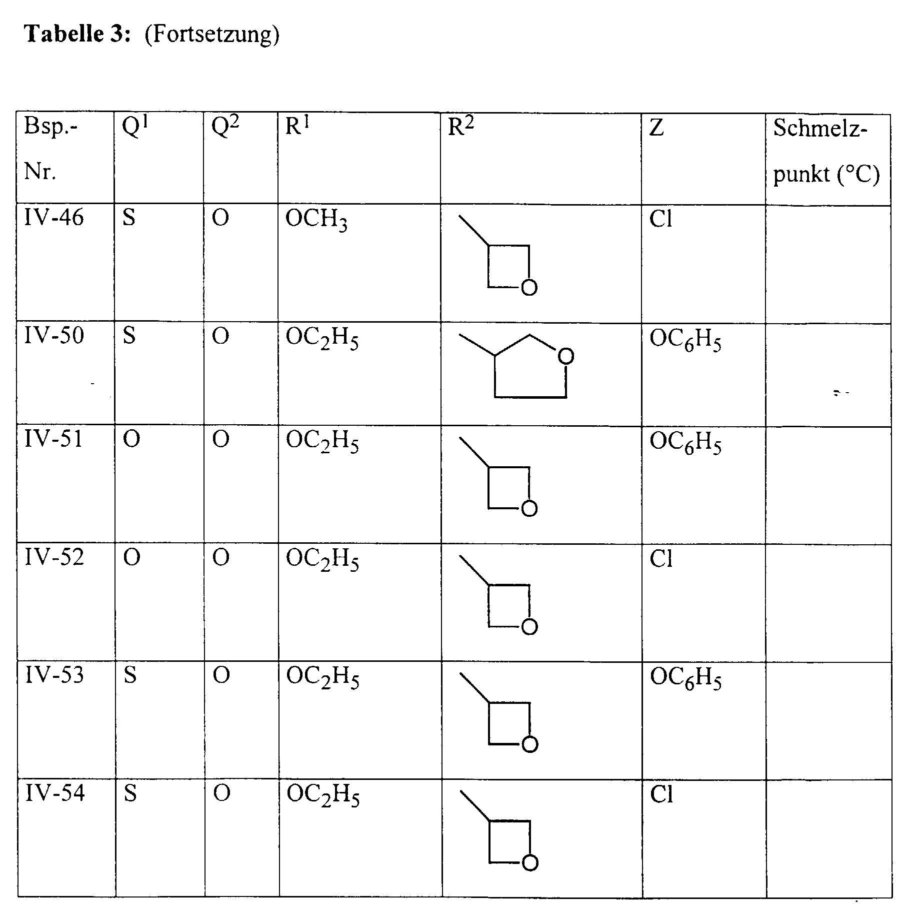

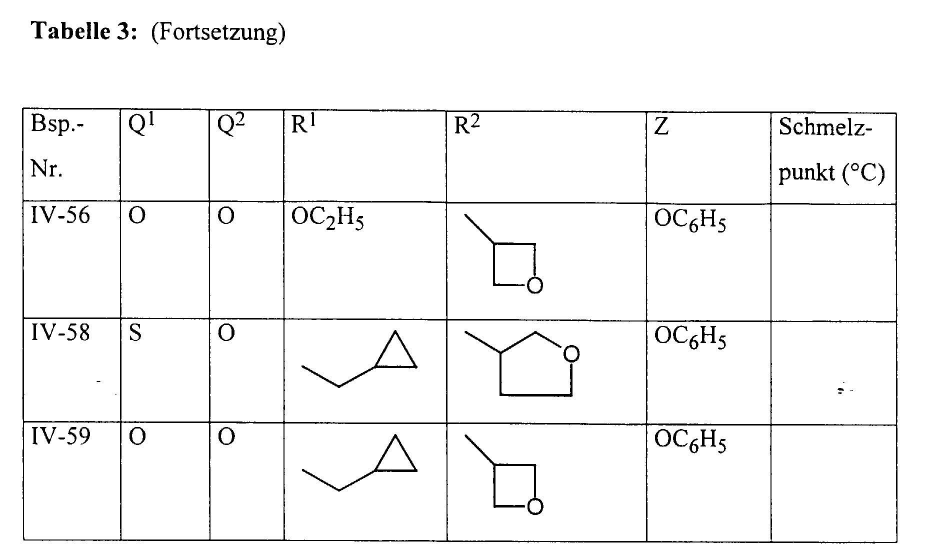

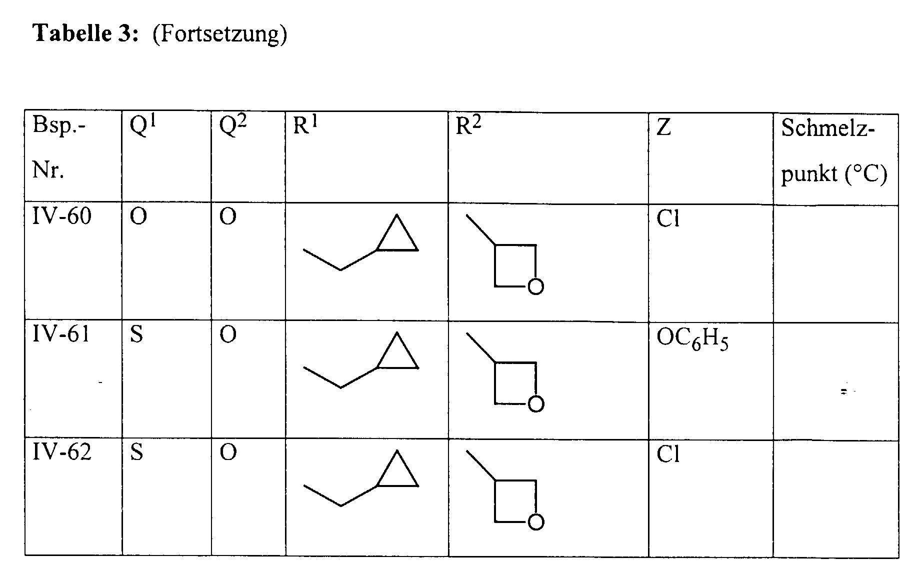

### Anwendungsbeispiele

### Beispiel A

| Pre-emergence-Test | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung
ai. = "active ingredient" = Wirkstoff

In diesem Test zeigen die erfindungsgemäßen Verbindungen der Formel (I) starke Wirkung gegen Unkräuter (vgl. Tabelle A).

### Beispiel B

| Post-emergence-Test | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen die erfindungsgemäßen Verbindungen der Formel (I) starke Wirkung gegen Unkräuter (vgl. Tabelle B).

**Tabelle A**

| Wirkstoff gemãß Herstell.-Bsp.-Nr. | Pre-emergence Test / Gewächshaus | | | | | | | | |
|---|---|---|---|---|---|---|---|---|---|
| | g ai./ha | Alopecurus | Bromus | Cyperus | Lolium | Amaranthus | Cassia | Chenopodium | Veronica |
| (34) (vgl. Tabelle 1) | 500 | 100 | 100 | 100 | 100 | 100 | 100 | 100 | 100 |

**Tabelle B**

| Wirkstoff gemäß Herstell-bsp. Nr. | Post-emergence Test / Gewächshaus | | | | |
|---|---|---|---|---|---|
| | g ai./ha | Amaranthus | Solanum | Stellaria | Xanthium |
| (34) (vgl. Tabelle 1) | 500 | 95 | 95 | 90 | 95 |

## Patentansprüche

1. Sulfonylamino(thio)carbonyltriazolinone mit Heterocyclyloxy-Substituenten der allgemeinen Formel (I), 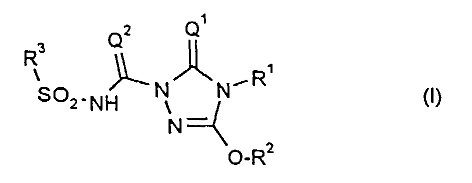 in welcher
Q¹ für Sauerstoff steht,
Q² für Sauerstoff oder Schwefel steht,
R¹ für Wasserstoff, Hydroxy, Amino, C₁-C₆-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für C₁-C₆-Alkyloxy oder C₃-C₆-Alkenyloxy, für jeweils gegebenenfalls durch Fluor und/oder Chlor substituiertes C₁-C₆-Alkylamino, Di-(C₁-C₄-alkyl)amino oder C₁-C₄-Alkanoylamino, für jeweils gegebenenfalls durch Fluor, Chlor, Brom und/oder C₁-C₄-Alkyl substituiertes C₃-C₆-Cycloalkyl oder C₃-C₆-Cycloalkyl-C₁-C₄-alkyl, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl oder Phenyl-C₁-C₄-alkyl steht,
R² für Oxetanyl oder Tetrahydrofuryl steht und
R³ für die Gruppierung 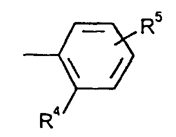 steht,
worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxycarbonyl, C₁-C₄-Alkylamino-carbonyl, Di-(C₁-C₄-alkyl)-amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxycarbonyl, C₁-C₄-Alkylthio, C₁-C₄-Alkylsulfinyl oder C₁-C₄-Alkylsulfonyl substituiert ist), für C₂-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxycarbonyl substituiert ist), C₃-C₆-Alkinyloxy, C₃-C₆-Alkinylthio oder für den Rest -S(O)ₚ-R⁶ stehen, wobei
p für die Zahlen 1 oder 2 steht und
R⁶ für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₁-C₄-Alkoxy-carbonyl, C₁-C₄-Alkylamino-carbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-Cycloalkyl-C₁-C₂-alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio,Trifluormethylthio oderC₁-C₄-Alkoxy-carbonyl substituiert ist) steht,
R⁴ und/oder R⁵ weiterhin für Phenyl oder Phenoxy, für C₁-C₄-Alkylcarbonylamino, C₁-C₄-Alkoxy-carbonylamino, C₁-C₄-Alkylamino-carbonyl-amino, Di-(C₁-C₄-alkyl)-amino-carbonylamino, oder für den Rest -CO-R⁸ stehen, wobei
R⁸ für Wasserstoff, C₁-C₆-Alkyl, C₃-C₆-Cycloalkyl, C₁-C₆-Alkoxy, C₃-C₆-Cycloalkoxy, C₃-C₆-Alkenyloxy, C₁-C₄-Alkylthio, C₁-C₄-Alkylamino, C₁-C₄-Alkoxyamino, C₁-C₄-Alkoxy-C₁-C₄-alkyl-amino oder Di-(C₁-C₄-alkyl)-amino steht (welche gegebenenfalls durch Fluor und/oder Chlor substituiert sind),
R⁴ und/oder R⁵ weiterhin für Trimethylsilyl, Thiazolinyl, C₁-C₄-Alkylsulfonyloxy, Di-(C₁-C₄-alkyl)-aminosulfonylamino oder für den Rest
-CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenyloxy, C₃-C₆-Alkinyloxy oder Benzyloxy, für Amino, C₁-C₄-Alkylamino, Di-(C₁-C₄-alkyl)-amino, Phenylamino, C₁-C₄-Alkyl-carbonylamino, C₁-C₄-Alkoxy-carbonylamino, C₁-C₄-Alkylsulfonylamino oder für gegebenenfalls durch Fluor, Chlor, Brom oder Methyl substituiertes Phenylsulfonylamino steht,
weiterhin
R³ für den Rest 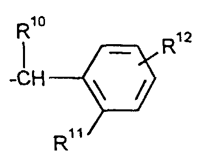 steht,
worin
R¹⁰ für Wasserstoff oder C₁-C₄-Alkyl steht,
R¹¹ und R¹² gleich oder verschieden sind und für Wasserstoff, Fluor, Chlor, Brom, Nitro, Cyano, C₁-C₄-Alkyl(welches gegebenenfalls durch Fluor und/oder Chlor substituiert ist), C₁-C₄-Alkoxy (welches gegebenenfalls durch Fluor und/oder Chlor substituiert ist), Carboxy, C₁-C₄-Alkoxy-carbonyl, Dimethylaminocarbonyl, C₁-C₄-Alkylsulfonyl oder Di-(C₁-C₄-alkyl)-aminosulfonyl stehen;
weiterhin
R³ für den Rest 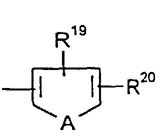 steht,
worin
R¹⁹ und R²⁰ gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), C₁-C₄-Alkylthio, C₁-C₄-Alkylsulfinyl oder C₁-C₄-Alkylsulfonyl (welche gegebenenfalls durch Fluor und/oder Chlor substituiert sind), Di-(C₁-C₄-alkyl)-amino-sulfonyl, C₁-C₄-Alkoxy-carbonyl oder Dimethylaminocarbonyl stehen, und
A für Schwefel steht,
sowie die Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium-, Tri-(C₁-C₄-alkyl)-sulfonium-, C₅- oder C₆-Cycloalkyl-ammonium- und Di-(C₁-C₂-alkyl)-benzyl-ammonium-Salze von Verbindungen der Formel (I).

2. Verbindungen der Formel (I) gemäß Anspruch 1, dadurch gekennzeichnet, daß darin
Q¹ für Sauerstoff steht,
Q² für Sauerstoff oder Schwefel steht,
R¹ für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für Methoxy, Ethoxy, n- oder i-Propoxy oder für Allyloxy, für Methylamino, Ethylamino, n- oder i-Propylamino, n-, i-, s- oder t-Butylamino, Dimethylamino oder Diethylamino, für jeweils gegebenenfalls durch Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Fluor, Chlor, Brom, Cyano, Methyl, Trifluormethyl oder Methoxy substituiertes Benzyl oder Phenyl steht,
R² für Oxetanyl oder Tetrahydrofuryl steht und
R³ für den Rest 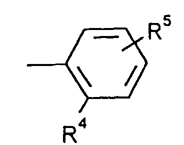 steht,
worin
R⁴ für Fluor, Chlor, Brom, Methyl, Ethyl, n- oder i-Propyl, Trifluormethyl, Methoxy, Ethoxy, n- oder i-Propoxy, n- oder i-Butoxy, Difluormethoxy, Trifluormethoxy, 2-Chlor-ethoxy, 2,2,2-Trifluor-ethoxy, 1,1,2,2-Tetrafluor-ethoxy, 1,1,2,2,2-Pentafluor-ethoxy, 2-Methoxy-ethoxy, Methylthio, Ethylthio, n- oder i-Propylthio, n- oder i-Butylthio, 2-Fluor-ethylthio, Allyloxy, Propargyloxy, Methylsulfinyl, Ethylsulfinyl, Methylsulfonyl, Ethylsulfonyl, Dimethylaminosulfonyl, Diethylaminosulfonyl, N-Methoxy-N-methylaminosulfonyl, Phenyl, Phenoxy, Methoxycarbonyl, Ethoxycarbonyl, n- oder i-Propoxycarbonyl steht und
R⁵ für Wasserstoff, Fluor, Chlor, Brom Methyl, Ethyl, n- oder i-Propyl, Trifluormethyl, Methoxy, Ethoxy, n- oder i-Propoxy, Difluormethoxy, Trifluormethoxy, Methylthio oder Ethylthio steht;
weiterhin
R³ für den Rest 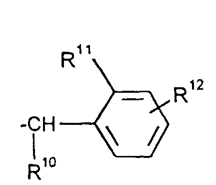 steht,
worin
R¹⁰ für Wasserstoff steht,
R¹¹ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
R¹² für Wasserstoff steht;
oder
R³ für den Rest 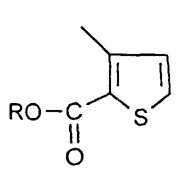 steht,
worin
R für Methyl, Ethyl, n- oder i-Propyl steht.

3. Verfahren zur Herstellung von Verbindungen der Formel (I) gemäß Anspruch 1, dadurch gekennzeichnet, daß man
(a) Triazolinone der allgemeinen Formel (II) 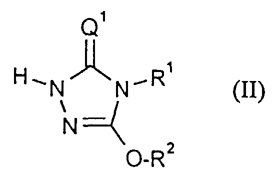 in welcher
Q¹, R¹ und R² die in Anspruch 1 angegebenen Bedeutungen haben,
mit Sulfonyliso(thio)cyanaten der allgemeinen Formel (III)
R³-SO₂-N=C=Q² (III)
in welcher
Q² und R³ die in Anspruch 1 angegebenen Bedeutungen haben,
gegebenenfalls in Gegenwart eines Reaktionshilfsmittels und gegebenenfalls in Gegenwart eines Verdünnungsmittels umsetzt,
oder daß man
(b) Triazolinon-Derivate der allgemeinen Formel (IV) 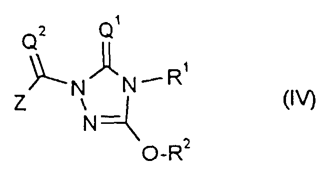 in welcher
Q¹, Q², R¹ und R² die oben angegebenen Bedeutungen haben und
Z für Fluor, Chlor, Brom, Methoxy, Ethoxy, Benzyloxy, Phenoxy, Halogen- oder Nitro-phenoxy steht,
mit Sulfonsäureamiden der allgemeinen Formel (V)
R³-SO₂-NH₂ (V)
in welcher
R³ die oben angegebene Bedeutung hat,
gegebenenfalls in Gegenwart eines Säureakzeptors und gegebenenfalls in Gegenwart eines Verdünnungsmittels umsetzt,
oder daß man
(c) Triazolinone der allgemeinen Formel (II) 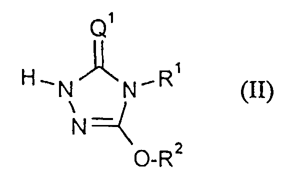 in welcher
Q¹, R¹ und R² die oben angegebenen Bedeutungen haben,
mit Sulfonsäureamid-Derivaten der allgemeinen Formel (VI)
R³-SO₂-NH-CQ²-Z (VI)
in welcher
Q² und R³ die oben angegebenen Bedeutungen haben und
Z für Fluor, Chlor, Brom, Methoxy, Ethoxy, Benzyloxy oder Phenoxy steht,
gegebenenfalls in Gegenwart eines Säureakzeptors und gegebenenfalls in Gegenwart eines Verdünnungsmittels umsetzt,
oder daß man
(d) Triazolinone der allgemeinen Formel (II) 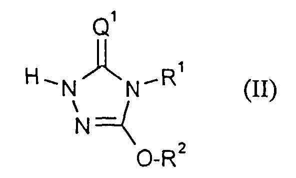 in welcher
Q¹, R¹ und R² die oben angegebenen Bedeutungen haben,
mit Sulfonsäurehalogeniden der allgemeinen Formel (VII)
R³-SO₂-X (VII)
in welcher
R³ die oben angegebene Bedeutung hat und
X für Halogen steht,
und Metall(thio)cyanaten der allgemeinen Formel (VIII)
MQ²CN (VIII)
in welcher
Q² die oben angegebene Bedeutung hat und
M für ein Alkalimetall oder Erdalkalimetall-äquivalent steht,
gegebenenfalls in Gegenwart eines Reaktionshilfsmittels und gegebenenfalls in Gegenwart eines Verdünnungsmittels umsetzt,
und gegebenenfalls die nach Verfahren (a), (b), (c) oder (d) erhaltenen Verbindungen der Formel (I) nach üblichen Methoden in Salze überführt.

4. Herbizide Mittel, gekennzeichnet, durch einen Gehalt an mindestens einer Verbindung der Formel (I) oder einem ihrer Salze gemäß Anspruch 1.

5. Verwendung von Verbindungen der allgemeinen Formel (I) oder deren Salzen gemäß Anspruch 1 zur Bekämpfung von unerwünschtem Pflanzenwachstum.

6. Verfahren zur Bekämpfung von Unkräutern, dadurch gekennzeichnet, daß man Verbindungen der allgemeinen Formel (I) oder deren Salze gemäß Anspruch 1 auf die Unkräuter oder ihren Lebensraum einwirken läßt.

7. Verfahren zur Herstellung von herbiziden Mitteln, dadurch gekennzeichnet, daß man Verbindungen der allgemeinen Formel (I) oder deren Salze gemäß Anspruch 1 mit Streckmitteln und/oder oberflächenaktiven Mitteln vermischt.

8. Triazolinone der allgemeinen Formel (II) 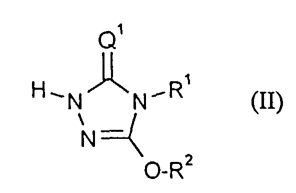 in welcher
Q¹ für Sauerstoff steht, und
R¹ und R² die in Anspruch 1 angegebenen Bedeutungen haben.

9. Triazolinon-Derivate der allgemeinen Formel (IV) 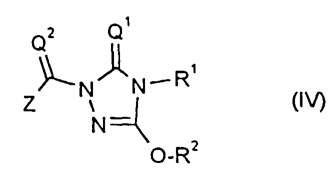 in welcher
Q¹ für Sauerstoff steht,
Q² für Sauerstoff oder Schwefel steht,
Z für Fluor, Chlor, Brom, Methoxy, Ethoxy, Benzyloxy, Phenoxy, Halogen- oder Nitro-phenoxy steht, und
R¹ und R² die in Anspruch 1 angegebenen Bedeutungen haben.

## Claims

1. Sulphonylamino(thio)carbonyltriazolinones having heterocyclyloxy substituents, of the general formula (I) 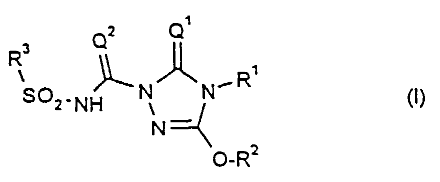 in which
Q¹ represents oxygen,
Q² represents oxygen or sulphur,
R¹ represents hydrogen, hydroxyl, amino or C₁-C₆-alkylideneamino, or represents optionally fluoro-, chloro-, bromo-, cyano-, C₁-C₄-alkoxy-, C₁-C₄-alkylcarbonyl- or C₁-C₄-alkoxy-carbonyl-substituted C₁-C₆-alkyl, or represents in each case optionally fluoro-, chloro- and/or bromo-substituted C₂-C₆-alkenyl or C₂-C₆-alkinyl, or represents C₁-C₆-alkyloxy or C₃-C₆-alkenyloxy, or represents in each case optionally fluoro- and/or chloro-substituted C₁-C₆-alkylamino, di-(C₁-C₄-alkyl)-amino or C₁-C₄-alkanoylamino, or represents in each case optionally fluoro-, chloro-, bromo- and/or C₁-C₄-alkyl-substituted C₃-C₆-cycloalkyl or C₃-C₆-cycloalkyl-C₁-C₄-alkyl, or represents in each case optionally fluoro-, chloro-, bromo-, cyano-, nitro-, C₁-C₄-alkyl-, trifluoromethyl-, C₁-C₄-alkoxy- and/or C₁-C₄-alkoxy-carbonyl-substituted phenyl or phenyl-C₁-C₄-alkyl,
R² represents oxetanyl or tetrahydrofuryl, and
R³ represents the group 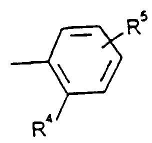 in which
R⁴ and R⁵ are identical or different and represent hydrogen, fluoro, chloro, bromo, iodo, nitro or C₁-C₆-alkyl (which is optionally substituted by fluoro, chloro, bromo, cyano, carboxyl, C₁-C₄-alkoxycarbonyl, C₁-C₄-alkylamino-carbonyl, di- (C₁-C₄-alkyl) -amino-carbonyl, hydroxyl, C₁-C₄-alkoxy, formyloxy, C₁-C₄-alkyl-carbonyloxy, C₁-C₄-alkoxy-carbonyloxy, C₁-C₄-alkylamino-carbonyloxy, C₁-C₄-alkylthio, C₁-C₄-alkylsulphinyl, C₁-C₄-alkylsulphonyl, di-(C₁-C₄-alkyl)-amino-sulphonyl, C₃-C₆-cycloalkyl or phenyl), or represent C₂-C₆-alkenyl (which is optionally substituted by fluoro, chloro, bromo, cyano, C₁-C₄-alkoxy-carbonyl, carboxyl or phenyl), or represent C₂-C₆-alkinyl (which is optionally substituted by fluoro, chloro, bromo, cyano, C₁-C₄-alkoxy-carbonyl, carboxyl or phenyl), or represent C₁-C₄-alkoxy (which is optionally substituted by fluoro, chloro, bromo, cyano, carboxyl, C₁-C₄-alkoxy-carbonyl, C₁-C₄-alkoxy, C₁-C₄-alkylthio, C₁-C₄-alkylsulphinyl or C₁-C₄-alkylsulphonyl), or represent C₁-C₄-alkylthio (which is optionally substituted by fluoro, chloro, bromo, cyano, carboxyl, C₁-C₄-alkoxy-carbonyl, C₁-C₄-alkylthio, C₁-C₄-alkylsulphinyl or C₁-C₄-alkylsulphonyl), or represent C₂-C₆-alkenyloxy (which is optionally substituted by fluoro, chloro, bromo, cyano or C₁-C₄-alkoxy-carbonyl), or represent C₂-C₆-alkenylthio (which is optionally substituted by fluoro, chloro, bromo, cyano, nitro, C₁-C₃-alkylthio or C₁-C₄-alkoxy-carbonyl), C₃-C₆-alkinyloxy or C₃-C₆-alkinylthio, or represent the radical -S(O)ₚ-R⁶, where
p represents the numbers 1 or 2 and
R⁶ represents C₁-C₄-alkyl (which is optionally substituted by fluoro, chloro, bromo, cyano or C₁-C₄-alkoxy-carbonyl), C₃-C₆-alkenyl, C₃-C₆-alkinyl, C₁-C₄-alkoxy, C₁-C₄-alkoxy-C₁-C₄-alkylamino, C₁-C₄-alkylamino, di-(C₁-C₄-alkyl)-amino or phenyl or represents the radical -NHOR⁷, where
R⁷ represents C₁-C₁₂-alkyl (which is optionally substituted by fluoro, chloro, cyano, C₁-C₄-alkoxy, C₁-C₄-alkylthio, C₁-C₄-alkylsulphinyl, C₁-C₄-alkylsulphonyl, C₁-C₄-alkyl-carbonyl, C₁-C₄-alkoxy-carbonyl, C₁-C₄-alkylamino-carbonyl or Di-(C₁-C₄-alkyl)-amino-carbonyl), or represents C₃-C₆-alkenyl (which is optionally substituted by fluoro, chloro or bromo), C₃-C₆-alkinyl, C₃-C₆-cycloalkyl, C₃-C₆-cycloalkyl-C₁-C₂-alkyl or phenyl-C₁-C₂-alkyl (which is optionally substituted by fluoro, chloro, nitro, cyano, C₁-C₄-alkyl, C₁-C₄-alkoxy or C₁-C₄-alkoxy-carbonyl), or represents benzhydryl or represents phenyl (which is optionally substituted by fluoro, chloro, nitro, cyano, C₁-C₄-alkyl, trifluoromethyl, C₁-C₄-alkoxy, C₁-C₂-fluoroalkoxy, C₁-C₄-alkylthio, trifluoromethylthio or C₁-C₄-alkoxy-carbonyl),
R⁴ and/or R⁵ additionally represent phenyl or phenoxy, or represent C₁-C₄-alkylcarbonylamino, C₁-C₄-alkoxy-carbonylamino, C₁-C₄-alkylamino-carbonylamino or di- (C₁-C₄-alkyl) -aminocarbonyl amino, or represent the radical -CO-R⁸, where
R⁸ represents hydrogen, C₁-C₆-alkyl, C₃-C₆-cycloalkyl, C₁-C₆-alkoxy, C₃-C₆-cycloalkoxy, C₃-C₆-alkenyloxy, C₁-C₄-alkylthio, C₁-C₄-alkylamino, C₁-C₄-alkoxyamino, C₁-C₄-alkoxy-C₁-C₄-alkylamino or di-(C₁-C₄-alkyl)-amino (which are optionally substituted by fluoro and/or chloro),
R⁴ and/or R⁵ additionally represent trimethylsilyl, thiazolinyl, C₁-C₄-alkyl-sulphonyloxy or di-(C₁-C₄-alkyl)-aminosulphonylamino or represent the radical
-CH=N-R⁹, where
R⁹ represents optionally fluoro-, chloro-, cyano-, carboxyl-, C₁-C₄-alkoxy-, C₁-C₄-alkylthio-, C₁-C₄-alkylsulphinyl- or C₁-C₄-alkylsulphonyl-substituted C₁-C₆-alkyl, or represents optionally fluoro- or chloro-substituted benzyl, or represents optionally fluoro- or chloro-substituted C₃-C₆-alkenyl or C₃-C₆-alkinyl, or represents optionally fluoro-, chloro-, bromo-, C₁-C₄-alkyl-, C₁-C₄-alkoxy-, trifluoromethyl-, trifluoromethoxy- or trifluoromethylthio-substituted phenyl, or represents optionally fluoro- and/or chloro-substituted C₁-C₆-alkoxy, C₃-C₆-alkenyloxy, C₃-C₆-alkinyloxy or benzyloxy, or represents amino, C₁-C₄-alkylamino, di-(C₁-C₄-alkyl)-amino, phenylamino, C₁-C₄-alkyl-carbonylamino, C₁-C₄-alkoxycarbonylamino or C₁-C₄-alkyl-sulphonylamino or represents optionally fluoro-, chloro-, bromo- or methyl-substituted phenyl-sulphonylamino,
and also
R³ represents the radical 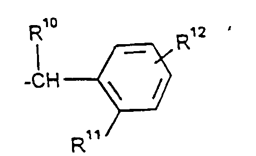 in which
R¹⁰ represents hydrogen or C₁-C₄-alkyl,
R¹¹ and R¹² are identical or different and represent hydrogen, fluoro, chloro, bromo, nitro, cyano, C₁-C₄-alkyl (which is optionally substituted by fluoro and/or chloro), C₁-C₄-alkoxy (which is optionally substituted by fluoro and/or chloro), carboxyl, C₁-C₄-alkoxy-carbonyl, dimethylaminocarbonyl, C₁-C₄-alkyl-sulphonyl or di-(C₁-C₄-alkyl)-aminosulphonyl;
and also
R³ represents the radical 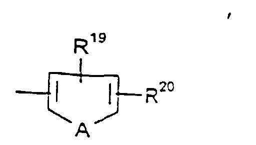 in which
R¹⁹ and R²⁰ are identical or different and represent hydrogen, fluoro, chloro, bromo, cyano, nitro, C₁-C₄-alkyl (which is optionally substituted by fluoro and/or chloro), C₁-C₄-alkoxy (which is optionally substituted by fluoro and/or chloro), C₁-C₄-alkylthio, C₁-C₄-alkylsulphinyl or C₁-C₄-alkylsulphonyl (which are optionally substituted by fluoro and/or chloro), di-(C₁-C₄-alkyl)-amino-sulphonyl, C₁-C₄-alkoxy-carbonyl or dimethylaminocarbonyl, and
A represents sulphur,
and also the sodium salts and the potassium, magnesium, calcium, ammonium, C₁-C₄-alkyl-ammonium, di-(C₁-C₄-alkyl)-ammonium, tri-(C₁-C₄-alkyl)-ammonium, tetra-(C₁-C₄-alkyl)-ammonium, tri-(C₁-C₄-alkyl)-sulphonium, C₅- or C₆-cycloalkyl-ammonium and di-(C₁-C₂-alkyl)-benzyl-ammonium salts of compounds of the formula (I).

2. Compounds of the formula (I) according to Claim 1, characterized in that in these compounds
Q¹ represents oxygen,
Q² represents oxygen or sulphur,
R¹ represents in each case optionally fluoro-, chloro-, cyano-, methoxy- or ethoxy-substituted methyl, ethyl, n- or i-propyl, n-, i-, s- or t-butyl, or represents in each case optionally fluoro-, chloro- or bromo-substituted propenyl, butenyl, propinyl or butinyl, or represents methoxy, ethoxy, n- or i-propoxy or represents allyloxy, or represents methylamino, ethylamino, n- or i-propylamino, n-, i-, s- or t-butylamino, dimethylamino or diethylamino, or represents in each case optionally fluoro-, chloro-, bromo-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl, or represents in each case optionally fluoro-, chloro-, bromo-, cyano-, methyl-, trifluoromethyl- or methoxy-substituted benzyl or phenyl,
R² represents oxetanyl, tetrahydrofuryl, and
R³ represents the radical 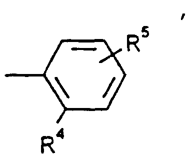 in which
R⁴ represents fluoro, chloro, bromo, methyl, ethyl, n- or i-propyl, trifluoromethyl, methoxy, ethoxy, n- or i-propoxy, n- or i-butoxy, difluoromethoxy, trifluoromethoxy, 2-chloro-ethoxy, 2,2,2-trifluoro-ethoxy, 1,1,2,2-tetrafluoroethoxy, 1,1,2,2,2-pentafluoro-ethoxy, 2-methoxy-ethoxy, methylthio, ethylthio, nor i-propylthio, n- or i-butylthio, 2-fluoro-ethylthio, allyloxy, propargyloxy, methylsulphinyl, ethylsulphinyl, methylsulphonyl, ethylsulphonyl, dimethylaminosulphonyl, diethylaminosulphonyl, N-methoxy-N-methyl-aminosulphonyl, phenyl, phenoxy, methoxycarbonyl, ethoxycarbonyl, n- or i-propoxycarbonyl, and
R⁵ represents hydrogen, fluoro, chloro, bromo methyl, ethyl, n- or i-propyl, trifluoromethyl, methoxy, ethoxy, n- or i-propoxy, difluoromethoxy, trifluoromethoxy, methylthio or ethylthio;
and also
R³ represents the radical 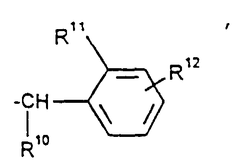 in which
R¹⁰ represents hydrogen,
R¹¹ represents fluoro, chloro, bromo, methyl, methoxy, difluoromethoxy, trifluoromethoxy, ethoxy, methoxycarbonyl, ethoxycarbonyl, methylsulphonyl or dimethylaminosulphonyl, and
R¹² represents hydrogen;
or
R³ represents the radical 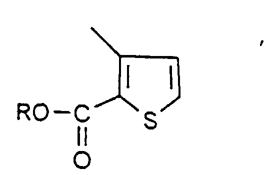 in which
R represents methyl, ethyl, n- or i-propyl.

3. Processes for the preparation of compounds of the formula (I) according to Claim 1, characterized in that
(a) triazolinones of the general formula (II) 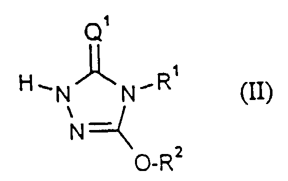 in which
Q¹, R¹ and R² have the meanings given in Claim 1
are reacted with sulphonyl iso(thio)cyanates of the general formula (III)
R³-SO₂-N=C=Q² (III)
in which
Q² and R³ have the meanings given in Claim 1, optionally in the presence of a reaction auxiliary and optionally in the presence of a diluent,
or
(b) triazolinone derivatives of the general formula (IV) 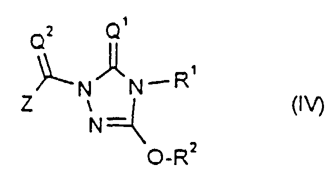 in which
Q¹, Q², R¹ and R² have the meanings given above
and
Z represents fluoro, chloro, bromo, methoxy, ethoxy, benzyloxy, phenoxy, halogeno- or nitro-phenoxy,
are reacted with sulphonamides of the general formula (V)
R³-SO₂-NH₂ (V)
in which
R³ has the meaning given above,
optionally in the presence of an acid acceptor and optionally in the presence of a diluent,
or
(c) triazolinones of the general formula (II) 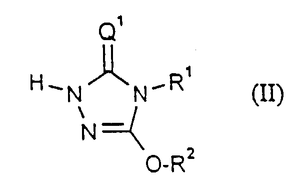 in which
Q¹, R¹ and R² have the meanings given above
are reacted with sulphonamide derivatives of the general formula (VI)
R³-SO₂-NH-CQ²-Z (VI)
in which
Q² and R³ have the meanings given above and
Z represents fluoro, chloro, bromo, methoxy, ethoxy, benzyloxy or phenoxy,
optionally in the presence of an acid acceptor and optionally in the presence of a diluent,
or
(d) triazolinones of the general formula (II) 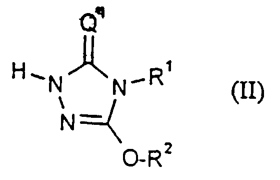 in which
Q¹, R¹ and R² have the meanings given above
are reacted with sulphonyl halides of the general formula (VII)
R³-SO₂-X (VII)
in which
R³ has the meaning given above and
X represents halogen
and with metal (thio)cyanates of the general formula (VIII)
MQ²CN (VIII)
in which
Q² has the meaning given above and
M represents an alkali metal or alkaline earth metal equivalent,
optionally in the presence of a reaction auxiliary and optionally in the presence of a diluent,
and if desired the compounds of the formula (I) obtained by process (a), (b), (c) or (d) are converted into salts by customary methods.

4. Herbicidal compositions, characterized, by a content of at least one compound of the formula (I) or one of its salts according to Claim 1.

5. Use of compounds of the general formula (I) or their salts according to Claim 1 for combating unwanted plant growth.

6. Method of combating weeds, characterized in that compounds of the general formula (I) or their salts according to Claim 1 are caused to act on the weeds or their habitat.

7. Process for the production of herbicidal compositions, characterized in that compounds of the general formula (I) or their salts according to Claim 1 are mixed with extenders and/or surface-active agents.

8. Triazolinones of the general formula (II) 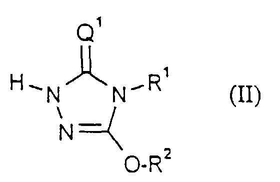 in which
Q¹ represents oxygen, and
R¹ and R² have the meanings given in Claim 1.

9. Triazolinone derivatives of the general formula (IV) 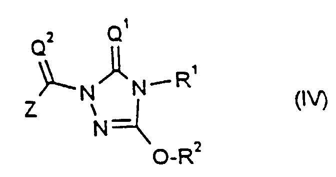 in which
Q¹ represents oxygen,
Q² represents oxygen or sulphur,
Z represents fluoro, chloro, bromo, methoxy, ethoxy, benzyloxy, phenoxy, halogeno- or nitro-phenoxy, and
R¹ and R² have the meanings given in Claim 1.

## Revendications

1. Sulfonylamino(thio)carbonyltriazolinones à substituants hétérocyclyloxy, de formule générale (I) 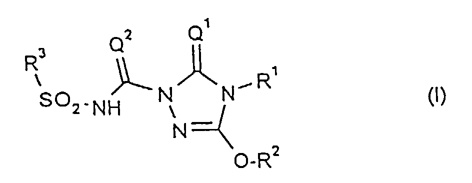 dans laquelle
Q¹ représente l'oxygène,
Q² représente l'oxygène ou le soufre,
R¹ est l'hydrogène, un groupe hydroxy, amino, alkylidène-amino en C₁ à C₆, un groupe alkyle en C₁ à C₆ éventuellement substitué par un radical fluoro, chloro, bromo, cyano, alkoxy en C₁ à C₄, (alkyle en C₁ à C₄)carbonyle ou (alkoxy en C₁ à C₄)carbonyle, un groupe alcényle en C₂ à C₆ ou alcynyle en C₂ à C₆ dont chacun est éventuellement substitué par du fluor, du chlore et/ou du brome, un groupe alkyloxy en C₁ à C₆ ou alcényloxy en C₃ à C₆, un groupe alkylamino en C₁ à C₆, di(alkyle en C₁ à C₄)amino ou alcanoylamino en C₁ à C₄ dont chacun est éventuellement substitué par du fluor et/ou du chlore, un groupe cycloalkyle en C₃ à C₆ ou (cycloalkyle en C₃ à C₆)-(alkyle en C₁ à C₄) dont chacun est substitué le cas échéant par un radical fluoro, chloro, bromo et/ou alkyle en C₁ à C₄, ou bien un groupe phényle ou phényl(alkyle en C₁ à C₄) dont chacun est substitué le cas échéant par un radical fluoro, chloro, bromo, cyano, nitro, alkyle en C₁ à C₄, trifluorométhyle, alkoxy en C₁ à C₄ et/ou (alkoxy en C₁ à C₄)carbonyle,
R² est un groupe oxétanyle ou tétrahydrofuryle et
R³ représente le groupement 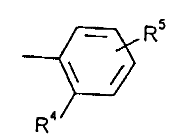 dans lequel
R⁴ et R⁵ sont identiques ou différents et représentent l'hydrogène, le fluor, le chlore, le brome, l'iode, un groupe nitro, alkyle en C₁ à C₆ (qui est substitué le cas échéant par un radical fluoro, chloro, bromo, cyano, carboxy, (alkoxy en C₁ à C₄) carbonyle, (alkylamino en C₁ à C₄)-carbonyle, di(alkyle en C₁ à C₄)aminocarbonyle, hydroxy, alkoxy en C₁ à C₄, formyloxy, (alkyle en C₁ à C₄)carbonyloxy, (alkoxy en C₁ à C₄)-carbonyloxy, (alkylamino en C₁ à C₄)carbonyloxy, alkylthio en C₁ à C₄, alkylsulfinyle en C₁ à C₄, alkylsulfonyle en C₁ à C₄, di(alkyle en C₁ à C₄)-aminosulfonyle, cycloalkyle en C₃ à C₆ ou phényle, un groupe alcényle en C₂ à C₆ (qui est substitué le cas échéant par le radical fluoro, chloro, bromo, cyano, (alkoxy en C₁ à C₄)carbonyle, carboxy ou phényle, un groupe alcynyle en C₂ à C₆, (qui est substitué le cas échéant par le radical fluoro, chloro, bromo, cyano, (alkoxy en C₁ à C₄)carbonyle, carboxy ou phényle, un groupe alkoxy en C₁ à C₄ (qui est substitué le cas échéant par le radical fluoro, chloro, bromo, cyano, carboxy, (alkoxy en C₁ à C₄)carbonyle, alkoxy en C₁ à C₄, alkylthio en C₁ à C₄, alkylsulfinyle en C₁ à C₄ ou alkylsulfonyle en C₁ à C₄), un groupe alkylthio en C₁ à C₄ (qui est substitué le cas échéant par le radical fluoro, chloro, bromo, cyano, carboxy, (alkoxy en C₁ à C₄)carbonyle, alkylthio en C₁ à C₄, alkylsulfinyle en C₁ à C₄ ou alkylsulfonyle en C₁ à C₄), un groupe alcényloxy en C₂ à C₆ (qui est substitué le cas échéant par le radical fluoro, chloro, bromo, cyano ou alkoxy en C₁ à C₄)carbonyle, un groupe alcénylthio en C₂ à C₆ (qui est substitué le cas échéant par le radical fluoro, chloro, bromo, cyano, nitro, alkylthio en C₁ à C₃ ou (alkoxy en C₁ à C₄) carbonyle, un groupe alcynyloxy en C₃ à C₆, alcynylthio en C₃ à C₆ ou le reste -S(O)ₚ-R⁶, dans lequel
p représente le nombre 1 ou 2 et
R⁶ est un groupe alkyle en C₁ à C₄ (qui est substitué le cas échéant par le radical fluoro, chloro, bromo, cyano ou (alkoxy en C₁ à C₄)carbonyle, un groupe alcényle en C₃ à C₆, alcynyle en C₃ à C₆, alkoxy en C₁ à C₄, (alkoxy en C₁ à C₄)-(alkylamino en C₁ à C₄), alkylamino en C₁ à C₄, di(alkyle en C₁ à C₄)-amino, phényle, ou le reste -NHOR⁷, dans lequel
R⁷ est un groupe alkyle en C₁ à C₁₂ (qui est substitué le cas échéant par le radical fluoro, chloro, cyano, alkoxy en C₁ à C₄, alkylthio en C₁ à C₄, alkylsulfinyle en C₁ à C₄, alkylsulfonyle en C₁ à C₄, (alkyle en C₁ à C₄)carbonyle, (alkoxy en C₁ à C₄)carbonyle, (alkylamino en C₁ à C₄)carbonyle ou di(alkyle en C₁ à C₄)aminocarbonyle, un groupe alcényle en C₃ à C₆ (qui est substitué le cas échéant par le radical fluoro, chloro ou bromo), un groupe alcynyle en C₃ à C₆, cycloalkyle en C₃ à C₆, (cycloalkyle en C₃ à C₆)-(alkyle en C₁ ou C₂), phényl(alkyle en C₁ ou C₂) (qui est substitué le cas échéant par le radical fluoro, chloro, nitro, cyano, alkyle en C₁ à C₄, alkoxy en C₁ à C₄ ou (alkoxy en C₁ à C₄)carbonyle, un groupe benzhydryle ou un groupe phényle (qui est substitué le cas échéant par le radical fluoro, chloro, nitro, cyano, alkyle en C₁ à C₄, trifluorométhyle, alkoxy en C₁ à C₄, trifluoralkoxy en C₁ ou C₂, alkylthio en C₁ à C₄, trifluorométhylthio ou (alkoxy en C₁ à C₄) carbonyle,
R⁴ et/ou R⁵ représentent en outre un groupe phényle ou phénoxy, un groupe (alkyle en C₁ à C₄)carbonylamino, (alkoxy en C₁ à C₄)-carbonylamino, (alkylamino en C₁ à C₄)-carbonylamino, di(alkyle en C₁ à C₄)-aminocarbonylamino ou le reste -CO-R⁸, dans lequel
R⁸ représente l'hydrogène, un groupe alkyle en C₁ à C₆, cycloalkyle en C₃ à C₆, alkoxy en C₁ à C₆, cycloalkoxy en C₃ à C₆, alcényloxy en C₃ à C₆, alkylthio en C₁ à C₄, alkylamino en C₁ à C₄, alkoxylamino en C₁ à C₄, (alkoxy en C₁ à C₄)-(alkylamino en C₁ à C₄) ou di(alkyle en C₁ à C₄)amino (ces groupes étant facultativement substitués par du fluor et/ou du chlore),
R⁴ et/ou R⁵ représentent en outre un groupe triméthylsilyle, thiazolinyle, alkylsulfonyloxy en C₁ à C₄, di(alkyle en C₁ à C₄)-aminosulfonylamino ou le reste
-CH=N-R⁹, dans lequel
R⁹ est un groupe alkyle en C₁ à C₆ éventuellement substitué par le radical fluoro, chloro, cyano, carboxy, alkoxy en C₁ à C₄, alkylthio en C₁ à C₄, alkylsulfinyle en C₁ à C₄ ou alkylsulfonyle en C₁ à C₄, un groupe benzyle éventuellement substitué par du fluor ou du chlore, un groupe alcényle en C₃ à C₆ ou alcynyle en C₃ à C₆ éventuellement substitué par du fluor ou du chlore, un groupe phényle éventuellement substitué par le radical fluoro, chloro, bromo, alkyle en C₁ à C₄, alkoxy en C₁ à C₄, trifluorométhyle, trifluorométhoxy ou trifluorométhylthio, un groupe alkoxy en C₁ à C₆ alcényloxy en C₃ à C₆, alcynyloxy en C₃ à C₆ ou benzyloxy éventuellement substitué par du fluor et/ou du chlore, un groupe amino, alkylamino en C₁ à C₄, di(alkyle en C₁ à C₄)amino, phénylamino, (alkyle en C₁ à C₄)carbonylamino, (alkoxy en C₁ à C₄)carbonylamino, alkylsulfonylamino en C₁ à C₄ ou un groupe phénylsulfonylamino éventuellement substitué par le radical fluoro, chloro, bromo ou méthyle,
en outre,
R³ représente le reste 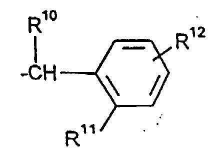 dans lequel
R¹⁰ est l'hydrogène ou un groupe alkyle en C₁ à C₄,
R¹¹ et R¹² sont identiques ou différents et représentent l'hydrogène, le fluor, le chlore, le brome, un groupe nitro, cyano, alkyle en C₁ à C₄ (qui est substitué le cas échéant par du fluor et/ou du chlore), un groupe alkoxy en C₁ à C₄ (qui est substitué le cas échéant par du fluor et/ou du chlore), un groupe carboxy, (alkoxy en C₁ à C₄)carbonyle, diméthylaminocarbonyle, alkylsulfonyle en C₁ à C₄ ou di(alkyle en C₁ à C₄) aminosulfonyle ;
en outre,
R³ représente le reste 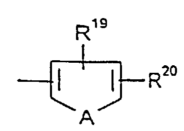 dans lequel
R¹⁹ et R²⁰ sont identiques ou différents et représentent l'hydrogène, le fluor, le chlore, le brome, un groupe cyano, nitro, alkyle en C₁ à C₄ (qui est substitué le cas échéant par du fluor et/ou du chlore), un groupe alkoxy en C₁ à C₄ (qui est substitué le cas échéant par du fluor et/ou du chlore), un groupe alkylthio en C₁ à C₄, alkylsulfinyle en C₁ à C₄ ou alkylsulfonyle en C₁ à C₄ (ces groupes étant substitués le cas échéant par du fluor et/ou du chlore), un groupe di(alkyle en C₁ à C₄)aminosulfonyle, (alkoxy en C₁ à C₄)carbonyle ou diméthylaminocarbonyle et
A représente le soufre,
ainsi que les sels de sodium, de potassium, de magnésium, de calcium, d'ammonium, d'(alkyle en C₁ à C₄)ammonium, de di(alkyle en C₁ à C₄)ammonium, de tri(alkyle en C₁ à C₄)-ammonium, de tétra(alkyle en C₁ à C₄)ammonium, de tri(alkyle en C₁ à C₄)sulfonium, de (cycloalkyle en C₅ ou C₆)ammonium et di(alkyle en C₁ ou C₂)benzylammonium de composés de formule (I).

2. Composés de formule (I) suivant la revendication 1, caractérisés en ce que, dans leur formule,
Q¹ représente l'oxygène,
Q² représente l'oxygène ou le soufre,
R¹ est un groupe méthyle, éthyle, n-propyle, isopropyle, n-butyle, isobutyle, sec.-butyle ou tertio-butyle éventuellement substitué dans chaque cas par le radical fluoro, chloro, cyano, méthoxy ou éthoxy, un groupe propényle, butényle, propinyle ou butynyle éventuellement substitué dans chaque cas par le radical fluoro, chloro ou bromo, un groupe méthoxy, éthoxy, n-propoxy, isopropoxy ou un groupe allyloxy, méthylamino, éthylamino, n-propylamino, isopropylmino, n-butylamino, isobutylamino, sec.-butylamino, tertio-butylamino, diméthylamino, ou diéthylamino, un groupe cyclopropyle, cyclobutyle, cyclopentyle, cyclohexyle, cyclopropylméthyle, cyclobutylméthyle ou cyclopentylméthyle ou cyclohexylméthyle éventuellement substitué dans chaque cas par le radical fluoro, chloro, bromo, méthyle ou éthyle, ou bien un groupe benzyle ou phényle dont chacun est éventuellement substitué par le radical fluoro, chloro, bromo, cyano, méthyle, trifluorométhyle ou méthoxy,
R² est un groupe oxétanyle ou tétrahydrofuryle et
R³ représente le reste 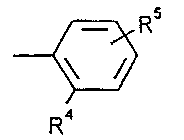 dans lequel
R⁴ est le fluor, le chlore, le brome, un groupe méthyle, éthyle, n-propyle, isopropyle, trifluorométhyle, méthoxy, éthoxy, n-propoxy, isopropoxy, n-butoxy, isobutoxy, difluorométhoxy, trifluorométhoxy, 2-chloréthoxy, 2,2,2-trifluoréthoxy, 1,1,2,2-tétrafluoréthoxy, 1,1,2,2,2-pentafluoréthoxy, 2-méthoxyéthoxy, méthylthio, éthylthio, n-propyl-thio, isopropylthio, n-butylthio, isobutylthio, 2-fluoréthylthio, allyloxy, propargyloxy, méthylsulfinyle, éthylsulfinyle, méthylsulfonyle, éthylsulfonyle, diméthylaminosulfonyle, diéthyl-aminosulfonyle, N-méthoxy-N-méthylaminosulfonyle, phényle, phénoxy, méthoxycarbonyle, éthoxy-carbonyle, n-propoxycarbonyle ou isopropoxy-carbonyle et
R⁵ représente l'hydrogène, le fluor, le chlore, le brome, un groupe méthyle, éthyle, n-propyle, isopropyle, trifluorométhyle, méthoxy, éthoxy, n-propoxy, isopropoxy, difluorométhoxy, trifluorométhoxy, méthylthio ou éthylthio ;
en outre
R³ représente le reste 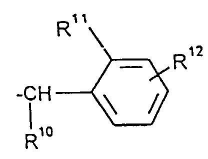 dans lequel
R¹⁰ est l'hydrogène,
R¹¹ représente le fluor, le chlore, le brome, un groupe méthyle, méthoxy, difluorométhoxy, trifluorométhoxy, éthoxy, méthoxycarbonyle, éthoxycarbonyle, méthylsulfonyle ou diméthylaminosulfonyle et
R¹² représente l'hydrogène ;
ou bien
R³ représente le reste 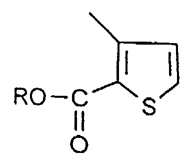 dans lequel
R est un groupe méthyle, éthyle, n-propyle ou isopropyle.

3. Procédé de production de composés de formule (I) suivant la revendication 1, caractérisé en ce que
(a) on fait réagir des triazolinones de formule générale (II) 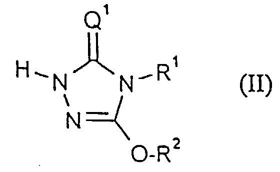 dans laquelle
Q¹, R¹ et R² ont les définitions indiquées dans la revendication 1,
avec des sulfonyliso(thio)cyanates de formule générale (III)
R³-SO₂-N=C=Q² (III)
dans laquelle
Q² et R³ ont les définitions indiquées dans la revendication 1,
le cas échéant en présence d'un auxiliaire de réaction et la présence éventuelle d'un diluant,
ou bien
(b) on fait réagir des dérivés de triazolinone de formule générale (IV) 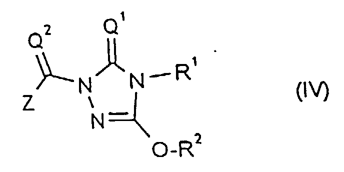 dans laquelle
Q¹, Q², R¹ et R² ont les définitions indiquées ci-dessus et
Z représente le fluor, le chlore, le brome, un groupe méthoxy, éthoxy, benzyloxy, phénoxy, halogénophénoxy ou nitrophénoxy,
avec des amides d'acide sulfonique de formule générale (V)
R³-SO₂-NH₂ (V)
dans laquelle
R³ a la définition indiquée ci-dessus,
le cas échéant en présence d'un accepteur d'acide et en la présence éventuelle d'un diluant,
ou bien
(c) on fait réagir des triazolinones de formule générale (II) 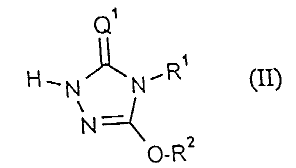 dans laquelle
Q, R¹ et R² on les définitions indiquées ci-dessus,
avec des dérivés de sulfonamides de formule générale (VI)
R³-SO₂-NH-CQ²-Z (VI)
dans laquelle
Q² et R³ ont les définitions indiquées ci-dessus et
Z représente le fluor, le chlore, le brome, un groupe méthoxy, éthoxy, benzyloxy ou phénoxy,
le cas échéant en présence d'un accepteur d'acide et en la présence éventuelle d'un diluant,
ou bien
(d) on fait réagir des triazolinones de formule générale (II) 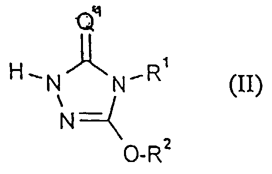 dans laquelle
Q¹, R¹ et R² ont les définitions indiquées ci-dessus,
avec des halogénures d'acide sulfonique de formule générale (VII)
R³-SO₂-X (VII)
dans laquelle
R³ a la définition indiquée ci-dessus et
X représente un halogène,
et des (thio)cyanates métalliques de formule générale (VIII)
MQ²CN (VIII)
dans laquelle
Q² a la définition indiquée ci-dessus et
M est un métal alcalin ou un équivalent de métal alcalino-terreux,
le cas échéant en présence d'un auxiliaire de réaction et en la présence éventuelle d'un diluant,
et on transforme éventuellement en sels par des modes opératoires classiques les composés de formule (I) obtenus selon le procédé (a), (b), (c) ou (d).

4. Compositions herbicides, caractérisées par une teneur en au moins un composé de formule (I) ou l'un de leurs sels suivant la revendication 1.

5. Utilisation de composés de formule générale (I) ou de leurs sels suivant la revendication 1 pour combattre la croissance de plantes indésirables.

6. Procédé pour combattre des mauvaises herbes, caractérisé en ce qu'on fait agir des composés de formule générale (I) ou leurs sels suivant la revendication 1 sur les mauvaises herbes ou sur leur milieu.

7. Procédé de préparation de compositions herbicides, caractérisé en ce qu'on mélange des composés de formule générale (I) ou leurs sels suivant la revendication 1 avec des diluants et/ou des agents tensio-actifs.

8. Triazolinones de formule générale (II) 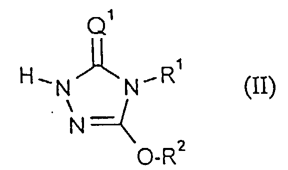 dans laquelle
Q¹ représente l'oxygène et
R¹ et R² ont les définitions indiquées dans la revendication 1.

9. Dérivés de triazolinones de formule générale (IV) 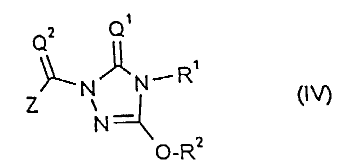 dans laquelle
Q¹ est l'oxygène,
Q² est l'oxygène ou le soufre,
Z représente le fluor, le chlore, le brome, un groupe méthoxy, éthoxy, benzyloxy, phénoxy, halogénophénoxy, ou nitrophénoxy et
R¹ et R² ont les définitions indiquées dans la revendication 1.
